(19) Europäisches Patentamt
European Patent Office
Office européen des brevets

(11) EP 1 541 562 A1

(12) **EUROPEAN PATENT APPLICATION**
published in accordance with Art. 158(3) EPC

(43) Date of publication:
**15.06.2005 Bulletin 2005/24**

(21) Application number: **03797642.0**

(22) Date of filing: **17.09.2003**

(51) Int Cl.[7]: **C07D 239/54**, C07D 405/06, C07D 405/12, C07D 405/14, A61K 31/513, A61P 17/04, A61P 37/08

(86) International application number:
**PCT/JP2003/011859**

(87) International publication number:
**WO 2004/026842 (01.04.2004 Gazette 2004/14)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **20.09.2002 JP 2002274559**

(71) Applicant: **Sumitomo Pharmaceuticals Company, Limited**
**Osaka 541-8510 (JP)**

(72) Inventors:
- **ISOBE, Yoshiaki**
  **c/o SUMITOMO PHARMACEUTIC. CO. Ltd**
  **Osaka-shi Osaka 554-0022 (JP)**
- **TOBE, Masanori**
  **c/o SUMITOMO PHARMACEUTIC. CO., Ltd**
  **Osaka-shi Osaka 554-0022 (JP)**
- **ISOBE, Masakazu**
  **c/o SUMITOMO PHARMACEUTIC. CO.,Ltd**
  **Osaka-shi, Osaka 554-0022 (JP)**
- **INOUE, Yoshifumi**
  **c/o SUMITOMO PHARMACEUTIC. CO.LTD**
  **Osaka-shi, Osaka 554-0022 (JP)**

(74) Representative: **Duckett, Anthony Joseph et al**
**J.A. Kemp & Co.,**
**14 South Square,**
**Gray's Inn**
**London WC1R 5JJ (GB)**

(54) **NOVEL URACIL DERIVATIVES AND MEDICINAL USE THEREOF**

(57) There are provided a uracil derivative represented by the general formula (I):

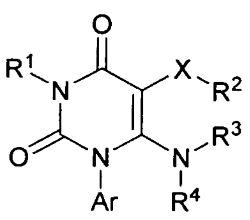

(I)

wherein X represents a group selected from NHCO, $NHCH_2$, CO, CONH and $CH_2NH$; $R^1$ represents a hydrogen atom or a substituted or unsubstituted alkyl group of 1 to 6 carbon atoms; $R^2$ represents the general formula (II) or (III) :

EP 1 541 562 A1

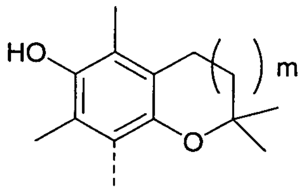

(II)

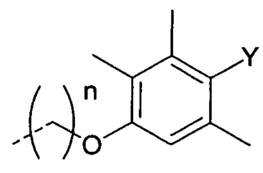

(III)

wherein m is 0 or 1, n is an integer of 1 to 3, Y is OH or $NH_2$, and each dotted line indicates a bonding position, provided that when $R^2$ represents the general formula (III), X represents NHCO or $NHCH_2$; $R^3$ and $R^4$ independently represent a hydrogen atom or an alkyl group of 1 to 6 carbon atoms; and Ar represents a phenyl group substituted by alkyl groups of 1 to 6 carbon atoms at the o- and m-positions, a substituted or unsubstituted heteroaryl group or a bicyclic aromatic group, or a pharmaceutically acceptable salt thereof, and a pharmaceutical composition for the treatment of, in particular, allergic diseases in which a type IV allergic reaction participates, which comprises the above-mentioned uracil derivative or pharmaceutically acceptable salt thereof as an active ingredient, that is, there are provided a novel compound useful for treating various allergic diseases, in particular, diseases in which a type IV allergic reaction participates, and a pharmaceutical composition for the treatment of allergic diseases comprising this compound as an active ingredient.

## Description

TECHNICAL FIELD

The present invention relates to novel uracil derivatives or pharmaceutically acceptable salts thereof and pharmaceutical compositions for the treatment of allergic diseases and pruritus which contain any of the uracil derivatives or pharmaceutically acceptable salts thereof as an active ingredient.

BACKGROUND ART

Allergic reactions capable of causing allergic diseases are mainly classified into type I to type IV allergic reactions. It is said that type IV allergic reactions play a large part in atopic dermatitis, contact dermatitis, chronic bronchial asthma, psoriasis, graft-versus-host disease and the like. The effectiveness of antihistamines and chemical mediator release inhibitors against these diseases is limited, and these diseases are treated by the use of steroids. In addition, immunosuprressive agents such as cyclosporin and tacrolimus are effective in suppressing rejection after organ transplantation or treating graft-versus-host disease. The employment of these drugs for the treatment of dermatitis is expanded and tacrolimus has already been clinically used for this treatment (Lancet, 339, 1120 (1992) and J. Invest. Dermatol., 98, 851 (1992)).

However, as to the steroids, their adverse effects such as infectious diseases, adrenal atrophy, osteoporosis, diabetes, failure to thrive of children, and the like are problems. Also in the case of the immunosuppressive agents such as cyclosporin and tacrolimus, the exhibition of adverse effects due to their immunosuppressive effect, such as infectious diseases, diabetes and the like is feared. Severe itch is known as one of the characteristics of the morbidity of atopic dermatitis and treatment thereof is clinically very important. However, the effectiveness of existing antihistamines, chemical mediator release inhibitors, steroids and immunosuppressive agents against the itch due to atopic dermatitis is low.

The present applicant has previously proposed uracil derivatives (see JP-A-8-109171) and hydroquinone derivatives (see Japanese Patent No. 3093170), which suppress type IV allergic reactions. There is still desired the development of a more effective therapeutic agent for allergic diseases, in particular, a therapeutic agent which permits suppression of diseases in which a type IV allergic reaction participates or itch accompanying these diseases.

DISCLOSURE OF THE INVENTION

In view of such a situation, an object of the present invention is to provide a novel compound which permits not only treatment of various allergic diseases, in particular, diseases in which a type IV allergic reaction participates, but also suppression of itch accompanying these diseases or suppression of nonallergic itch; and a pharmaceutical composition for the treatment of allergic diseases containing said compound as an active ingredient.

In recognition of such a background, in order to develop a therapeutic agent that is more effective against various allergic diseases, in particular, diseases in which a type IV allergic reaction participates, and that can suppress itch accompanying these diseases or nonallergic itch, the present inventors earnestly investigated an aryl group bonded to the 1-position of the uracil skeleton of the uracil derivative disclosed in JP-A-8-109171 and a substituent bonded to the 5-position of this uracil skeleton, and consequently found that the uracil derivative described below in which the aforesaid aryl group is a phenyl group substituted by alkyl groups of 1 to 6 carbon atoms at the o- and m-positions, a substituted or unsubstituted heteroaryl group or a bicyclic aromatic group, and which has a hydroquinone structure with antioxidant action or an structure analogous thereto, which is bonded to the 5-position by a suitable connecting group, suppresses type IV allergic reactions remarkably and also can suppress itch, whereby the present invention has been accomplished.

That is, the present invention includes the following aspects of the invention.

(1) A uracil derivative represented by the general formula (I):

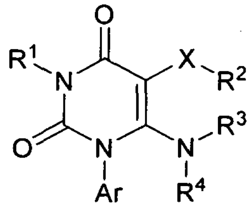

(I)

wherein X represents a group selected from NHCO, $NHCH_2$, CO, CONH and $CH_2NH$; $R^1$ represents a hydrogen atom or a substituted or unsubstituted alkyl group of 1 to 6 carbon atoms; $R^2$ represents the general formula (II) or (III):

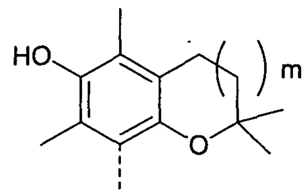

(II)

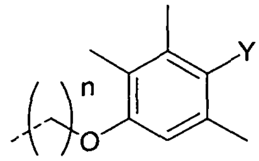

(III)

wherein m is 0 or 1, n is an integer of 1 to 3, Y is OH or $NH_2$, and each dotted line indicates a bonding position, provided that when $R^2$ represents the general formula (III), X represents NHCO or $NHCH_2$; $R^3$ and $R^4$ independently represent a hydrogen atom or an alkyl group of 1 to 6 carbon atoms; and Ar represents a phenyl group substituted by alkyl groups of 1 to 6 carbon atoms at the o- and m-positions, a substituted or substituted heteroaryl group or a bicyclic aromatic group,

or a pharmaceutically acceptable salt thereof.

(2) A uracil derivative or a pharmaceutically acceptable salt thereof according to the above item (1), wherein $R^2$ represents the general formula (II) in the general formula (I).

(3) A uracil derivative or a pharmaceutically acceptable salt thereof according to the above item (1), wherein $R^2$ represents the general formula (III) in the general formula (I).

(4) A uracil derivative or a pharmaceutically acceptable salt thereof according to the above item (1) or (2), wherein Ar represents a phenyl group substituted by alkyl groups of 1 to 6 carbon atoms at the o- and m-positions in the general formula (I).

(5) A uracil derivative or a pharmaceutically acceptable salt thereof according to the above item (1) or (2), wherein Ar represents a substituted or unsubstituted heteroaryl group in the general formula (I).

(6) A uracil derivative or a pharmaceutically acceptable salt thereof according to the above item (1) or (2), wherein Ar represents a bicyclic aromatic group in the general formula (I).

(7) A uracil derivative or a pharmaceutically acceptable salt thereof according to any one of the above items (1) to (6), wherein X represents NHCO in the general formula (I).

(8) A pharmaceutical composition for the treatment of allergic diseases comprising a uracil derivative or a pharmaceutically acceptable salt thereof according to any one of the above items (1) to (7) as an active ingredient.

(9) A pharmaceutical composition for the treatment of pruritus comprising a uracil derivative or a pharmaceutically acceptable salt thereof according to any one of the above items (1) to (7) as an active ingredient.

BEST MODE FOR CARRYING OUT THE INVENTION

In the present specification, the term "alkyl group of 1 to 6 carbon atoms" means a linear or branched alkyl group of 1 to 6 carbon atoms. Specific examples thereof are methyl group, ethyl group, propyl group (1-propyl group), isopropyl group (2-propyl group), butyl group (1-butyl group), sec-butyl group (2-butyl group), isobutyl group (2-methyl-1-propyl group), t-butyl group (2-methyl-2-propyl group), pentyl group (1-pentyl group) and hexyl group (1-hexyl group). Of these, preferable examples thereof are alkyl groups of 1 to 4 carbon atoms.

In the present specification, the term "alkoxy group of 1 to 6 carbon atoms" means a linear or branched alkoxy group of 1 to 6 carbon atoms. Specific examples thereof are methoxy group, ethoxy group, propoxy group, 1-methylethoxy group, butoxy group, 1-methylpropoxy group, 2-methylpropoxy group, 1,1-dimethylethoxy group, pentyloxy group and hexyloxy group. Of these, preferable examples thereof are alkoxy groups of 1 to 4 carbon atoms.

In the present specification, the halogen atom includes fluorine atom, chlorine atom, bromine atom and iodine atom.

The compound of the present invention is explained below in further detail. When $R^2$ represents the formula (II), X is a connecting group selected from NHCO, $NHCH_2$, CO, CONH and $CH_2NH$ and is preferably NHCO. When $R^2$ represents the formula (III), X is NHCO or $NHCH_2$.

Preferable examples of the alkyl group of 1 to 6 carbon atoms represented by $R^1$ are methyl group, ethyl group, propyl group (1-propyl group), isopropyl group (2-propyl group), butyl group (1-butyl group), sec-butyl group (2-butyl group), isobutyl group (2-methyl-1-propyl group) and t-butyl group (2-methyl-2-propyl group).

When the alkyl group of 1 to 6 carbon atoms represented by $R^1$ is a substituted alkyl group, its substituent(s)

includes halogen atoms, hydroxyl group and alkoxy groups of 1 to 6 carbon atoms. The alkyl group may have 1 to 3 substituents which may be the same or different. Specific examples of the substituents are hydroxyl group, methoxy group, ethoxy group and fluorine atom.

Preferable examples of $R^1$ are hydrogen atom, methyl group, ethyl group, propyl group (1-propyl group), butyl group (1-butyl group), 2-methoxyethyl group, 2-ethoxyethyl group, 3-methoxypropyl group, 2-hydroxyethyl group, 3-hydroxypropyl group, 4-hydrocybutyl group and 2,2,2-trifluoroethyl group. Of these, hydrogen atom, methyl group, ethyl group, propyl group, butyl group, 2-methoxyethyl group, 2-hydroxyethyl group and 3-hydroxypropyl group are especially preferable.

Preferable examples of each of $R^3$ and $R^4$ are hydrogen atom, methyl group, ethyl group and propyl group (1-propyl group). Of these, hydrogen atom and methyl group are especially preferable.

In the phenyl group substituted by alkyl groups of 1 to 6 carbon atoms at the o- and m-positions for Ar, the alkyl groups may be the same or different. Preferable examples of the phenyl group are 2,3-dimethylphenyl group, 2,3-diethylphenyl group, 2,3-dipropylphenyl group, 2,3-dibutylphenyl group, 2-ethyl-3-methylphenyl group, 2-methyl-3-ethylphenyl group and 2-methyl-3-propylphenyl group. Of these, 2,3-dimethylphenyl group is preferable.

As the heteroaryl group for Ar, 5- or 6-membered heteroaryl groups containing one or two nitrogen atoms are exemplified. Specific examples thereof are 2-pyrrolyl group, 3-pyrrolyl group, 2-pyridyl group, 3-pyridyl group, 4-pyridyl group, pyrimidinyl group and pyrazinyl group. Of these, 2-pyridyl group, 3-pyridyl group and 4-pyridyl group are preferable.

When the heteroaryl group is a substituted group, its substituent(s) includes alkyl groups of 1 to 6 carbon atoms, halogen atoms and alkoxy groups of 1 to 6 carbon atoms. Preferable examples of the aforesaid halogen atoms are fluorine atom and chlorine atom. Preferable examples of the aforesaid alkoxy group are methoxy group, ethoxy group, propoxy group, 1-methylethoxy group, butoxy group, 1-methylpropoxy group, 2-methylpropoxy group and 1,1-dimethylethoxy group.

Specific examples of the substituted heteroaryl group are 6-methyl-3-pyridyl group, 6-methoxy-3-pyridyl group, 6-chloro-3-pyridyl group, 2-methyl-3-pyridyl group, 2-methoxy-3-pyridyl group, 2-chloro-3-pyridyl group, 2,6-dimethyl-3-pyridyl group, 2,6-dimethoxyl-3-pyridyl group, 2,6-dichloro-3-pyridyl group and 2,3-dimethyl-4-pyridyl group. Of these, 2-methyl-3-pyridyl group is preferable.

As the bicyclic aromatic group, there are exemplified 9- or 10-membered bicyclic aromatic groups that may contain 1 to 3 heteroatoms selected from nitrogen atoms, oxygen atoms and sulfur atoms so that the number of nitrogen atoms may be 0 to 3, the number of oxygen atoms 0 to 2 and the number of sulfur atoms 0 or 1.

The bicyclic aromatic group preferably refers to a group represented by the formula (IV):

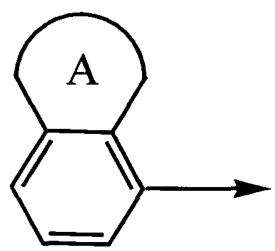

(IV)

wherein A is a 5- or 6-membered saturated or unsaturated carbocycle or heterocycle containing 0 to 2 heteroatoms selected from nitrogen atoms, oxygen atoms and sulfur atoms so that the number of nitrogen atoms may be 0 to 2, the number of oxygen atoms 0 to 2 and the number of sulfur atoms 0 to 2.

Specific examples of the bicyclic aromatic group are 1-naphthyl group, 5,6,7,8-tetrahydro-1-naphthyl group, 2,3-methylenedioxyphenyl group, 2,3-ethylenedioxyphenyl group, 4-indanyl group, 5-quinolyl group, 5-isoquinolyl group, 5-quinazolinyl group, 5-quinoxalinyl group, 4-benzofuranyl group, 4-benzothienyl group, 4-indazolyl group, 4-benzimidazolyl group and 4-benzothiazolyl group. Of these, 1-naphthyl group, 5,6,7,8-tetrahydro-1-naphthyl group, 4-indanyl group and 2,3-methylenedioxyphenyl group are preferable.

Specific preferable examples of the uracil derivative of the present invention are listed in Table 1 to Table 18.

In the following tables, $R^1$, $R^2$, X, Ar, m, n and Y are as defined in the general formula (I). In this case, each of $R^3$ and $R^4$ in the general formula (I) is hydrogen.

Table 1

| Compound | $R^1$ | $R^2$ | X | Ar | m or n | Y |
|---|---|---|---|---|---|---|
| 1 | Me | (II) | NHCO | 1-naphthyl | 0 | - |
| 2 | Me | (II) | NHCO | 1-naphthyl | 1 | - |
| 3 | Me | (III) | NHCO | 1-naphthyl | 1 | OH |
| 4 | Me | (III) | NHCO | 1-naphthyl | 1 | $NH_2$ |
| 5 | Me | (III) | NHCO | 1-naphthyl | 2 | OH |
| 6 | Me | (III) | NHCO | 1-naphthyl | 2 | $NH_2$ |
| 7 | Me | (III) | NHCO | 1-naphthyl | 3 | OH |
| 8 | Me | (III) | NHCO | 1-naphthyl | 3 | $NH_2$ |
| 9 | H | (II) | NHCO | 1-naphthyl | 0 | - |
| 10 | H | (II) | NHCO | 1-naphthyl | 1 | - |
| 11 | H | (III) | NHCO | 1-naphthyl | 1 | $NH_2$ |
| 12 | Et | (III) | NHCO | 1-naphthyl | 0 | $NH_2$ |
| 13 | Pr | (III) | NHCO | 1-naphthyl | 0 | $NH_2$ |
| 14 | Bu | (III) | NHCO | 1-naphthyl | 0 | $NH_2$ |
| 15 | Me | (II) | $NHCH_2$ | 1-naphthyl | 0 | - |
| 16 | Me | (II) | CONH | 1-naphthyl | 0 | - |
| 17 | Me | (II) | CO | 1-naphthyl | 0 | - |
| 19 | Et | (II) | NHCO | 1-naphthyl | 0 | - |
| 20 | Pr | (II) | NHCO | 1-naphthyl | 0 | - |
| 21 | Bu | (II) | NHCO | 1-naphthyl | 0 | - |
| 22 | Pr | (III) | NHCO | 1-naphthyl | 1 | OH |
| 23 | Pr | (III) | NHCO | 1-naphthyl | 0 | $NH_2$ |
| 24 | Pr | (III) | NHCO | 1-naphthyl | 1 | $NH_2$ |
| 25 | Pr | (III) | NHCO | 1-naphthyl | 0 | OH |
| 27 | Pr | (III) | NHCO | 1-naphthyl | 1 | $NH_2$ |
| 28 | Pr | (II) | CONH | 1-naphthyl | 0 | - |
| 29 | Pr | (II) | $NHCH_2$ | 1-naphthyl | 0 | - |
| 30 | Pr | (II) | CO | 1-naphthyl | 0 | - |

Table 2

| Compound | $R^1$ | $R^2$ | X | Ar | m or n | Y |
|---|---|---|---|---|---|---|
| 31 | H | (II) | NHCO | 4-indanyl | 0 | - |
| 32 | H | (II) | NHCO | 4-indanyl | 1 | - |
| 33 | H | (III) | NHCO | 4-indanyl | 1 | OH |
| 34 | H | (III) | NHCO | 4-indanyl | 1 | $NH_2$ |
| 35 | H | (III) | NHCO | 4-indanyl | 2 | OH |
| 36 | H | (III) | NHCO | 4-indanyl | 2 | $NH_2$ |
| 37 | H | (III) | NHCO | 4-indanyl | 3 | OH |

Table 2 (continued)

| Compound | $R^1$ | $R^2$ | X | Ar | m or n | Y |
|---|---|---|---|---|---|---|
| 38 | H | (III) | NHCO | 4-indanyl | 3 | $NH_2$ |
| 39 | H | (II) | $NHCH_2$ | 4-indanyl | 0 | - |
| 40 | H | (II) | $NHCH_2$ | 4-indanyl | 1 | - |
| 41 | H | (III) | $NHCH_2$ | 4-indanyl | 1 | OH |
| 42 | H | (III) | $NHCH_2$ | 4-indanyl | 1 | $NH_2$ |
| 43 | H | (III) | $NHCH_2$ | 4-indanyl | 2 | OH |
| 44 | H | (III) | $NHCH_2$ | 4-indanyl | 2 | $NH_2$ |
| 45 | H | (III) | $NHCH_2$ | 4-indanyl | 3 | OH |
| 46 | H | (III) | $NHCH_2$ | 4-indanyl | 3 | $NH_2$ |
| 47 | H | (II) | CONH | 4-indanyl | 0 | - |
| 48 | H | (II) | CONH | 4-indanyl | 1 | - |
| 49 | H | (II) | $CH_2NH$ | 4-indanyl | 0 | - |
| 50 | H | (II) | $CH_2NH$ | 4-indanyl | 1 | - |
| 51 | H | (II) | CO | 4-indanyl | 0 | - |
| 52 | H | (II) | NHCO | 4-indanyl | 0 | - |
| 53 | H | (III) | NHCO | 4-indanyl | 1 | OH |
| 54 | H | (III) | NHCO | 4-indanyl | 1 | $NH_2$ |
| 55 | H | (II) | NHCO | 4-indanyl | 0 | - |
| 56 | H | (III) | NHCO | 4-indanyl | 1 | $NH_2$ |
| 57 | H | (II) | NHCO | 4-indanyl | 0 | - |
| 58 | H | (III) | NHCO | 4-indanyl | 1 | $NH_2$ |
| 59 | H | (II) | NHCO | 4-indanyl | 0 | - |
| 60 | H | (III) | NHCO | 4-indanyl | 1 | $NH_2$ |

Table 3

| Compound | $R^1$ | $R^2$ | X | Ar | m or n | Y |
|---|---|---|---|---|---|---|
| 61 | Me | (II) | NHCO | 4-indanyl | 0 | - |
| 62 | Me | (II) | NHCO | 4-indanyl | 1 | - |
| 63 | Me | (III) | NHCO | 4-indanyl | 1 | OH |
| 64 | Me | (III) | NHCO | 4-indanyl | 1 | $NH_2$ |
| 65 | Me | (III) | NHCO | 4-indanyl | 2 | OH |
| 66 | Me | (III) | NHCO | 4-indanyl | 2 | $NH_2$ |
| 67 | Me | (III) | NHCO | 4-indanyl | 3 | OH |
| 68 | Me | (III) | NHCO | 4-indanyl | 3 | $NH_2$ |
| 69 | Me | (II) | $NHCH_2$ | 4-indanyl | 0 | - |
| 70 | Me | (II) | $NHCH_2$ | 4-indanyl | 1 | - |
| 71 | Me | (III) | $NHCH_2$ | 4-indanyl | 1 | OH |
| 72 | Me | (III) | $NHCH_2$ | 4-indanyl | 1 | $NH_2$ |

Table 3 (continued)

| Compound | $R^1$ | $R^2$ | X | Ar | m or n | Y |
|---|---|---|---|---|---|---|
| 73 | Me | (III) | $NHCH_2$ | 4-indanyl | 2 | OH |
| 74 | Me | (III) | $NHCH_2$ | 4-indanyl | 2 | $NH_2$ |
| 75 | Me | (III) | $NHCH_2$ | 4-indanyl | 3 | OH |
| 76 | Me | (III) | $NHCH_2$ | 4-indanyl | 3 | $NH_2$ |
| 77 | Me | (II) | CONH | 4-indanyl | 0 | - |
| 78 | Me | (II) | CONH | 4-indanyl | 1 | - |
| 79 | Me | (II) | $CH_2NH$ | 4-indanyl | 0 | - |
| 80 | Me | (II) | $CH_2NH$ | 4-indanyl | 1 | - |
| 81 | Me | (II) | CO | 4-indanyl | 0 | - |
| 82 | Me | (II) | NHCO | 4-indanyl | 0 | - |
| 83 | Me | (III) | NHCO | 4-indanyl | 1 | OH |
| 84 | Me | (III) | NHCO | 4-indanyl | 1 | $NH_2$ |
| 85 | Me | (II) | NHCO | 4-indanyl | 0 | - |
| 86 | Me | (III) | NHCO | 4-indanyl | 1 | $NH_2$ |
| 87 | Me | (II) | NHCO | 4-indanyl | 0 | - |
| 88 | Me | (III) | NHCO | 4-indanyl | 1 | $NH_2$ |
| 89 | Me | (II) | NHCO | 4-indanyl | 0 | - |
| 90 | Me | (III) | NHCO | 4-indanyl | 1 | $NH_2$ |

Table 4

| Compound | $R^1$ | $R^2$ | X | Ar | m or n | Y |
|---|---|---|---|---|---|---|
| 91 | Et | (II) | NHCO | 4-indanyl | 0 | - |
| 92 | Et | (II) | NHCO | 4-indanyl | 1 | - |
| 93 | Et | (III) | NHCO | 4-indanyl | 1 | OH |
| 94 | Et | (III) | NHCO | 4-indanyl | 1 | $NH_2$ |
| 95 | Et | (III) | NHCO | 4-indanyl | 2 | OH |
| 96 | Et | (III) | NHCO | 4-indanyl | 2 | $NH_2$ |
| 97 | Et | (III) | NHCO | 4-indanyl | 3 | OH |
| 98 | Et | (III) | NHCO | 4-indanyl | 3 | $NH_2$ |
| 99 | Et | (II) | $NHCH_2$ | 4-indanyl | 0 | - |
| 100 | Et | (II) | $NHCH_2$ | 4-indanyl | 1 | - |
| 101 | Et | (III) | $NHCH_2$ | 4-indanyl | 1 | OH |
| 102 | Et | (III) | $NHCH_2$ | 4-indanyl | 1 | $NH_2$ |
| 103 | Et | (III) | $NHCH_2$ | 4-indanyl | 2 | OH |
| 104 | Et | (III) | $NHCH_2$ | 4-indanyl | 2 | $NH_2$ |
| 105 | Et | (III) | $NHCH_2$ | 4-indanyl | 3 | OH |
| 106 | Et | (III) | $NHCH_2$ | 4-indanyl | 3 | $NH_2$ |
| 107 | Et | (II) | CONH | 4-indanyl | 0 | - |

Table 4 (continued)

| Compound | $R^1$ | $R^2$ | X | Ar | m or n | Y |
|---|---|---|---|---|---|---|
| 108 | Et | (II) | CONH | 4-indanyl | 1 | - |
| 109 | Et | (II) | $CH_2NH$ | 4-indanyl | 0 | - |
| 110 | Et | (II) | $CH_2NH$ | 4-indanyl | 1 | - |
| 111 | Et | (II) | CO | 4-indanyl | 0 | - |
| 112 | Et | (II) | NHCO | 4-indanyl | 0 | - |
| 113 | Et | (III) | NHCO | 4-indanyl | 1 | OH |
| 114 | Et | (III) | NHCO | 4-indanyl | 1 | $NH_2$ |
| 115 | Et | (II) | NHCO | 4-indanyl | 0 | - |
| 116 | Et | (III) | NHCO | 4-indanyl | 1 | $NH_2$ |
| 117 | Et | (II) | NHCO | 4-indanyl | 0 | - |
| 118 | Et | (III) | NHCO | 4-indanyl | 1 | $NH_2$ |
| 119 | Et | (II) | NHCO | 4-indanyl | 0 | - |
| 120 | Et | (III) | NHCO | 4-indanyl | 1 | $NH_2$ |

Table 5

| Compound | $R^1$ | $R^2$ | X | Ar | m or n | Y |
|---|---|---|---|---|---|---|
| 121 | Pr | (II) | NHCO | 4-indanyl | 0 | - |
| 122 | Pr | (II) | NHCO | 4-indanyl | 1 | - |
| 123 | Pr | (III) | NHCO | 4-indanyl | 1 | OH |
| 124 | Pr | (III) | NHCO | 4-indanyl | 1 | $NH_2$ |
| 125 | Pr | (III) | NHCO | 4-indanyl | 2 | OH |
| 126 | Pr | (III) | NHCO | 4-indanyl | 2 | $NH_2$ |
| 127 | Pr | (III) | NHCO | 4-indanyl | 3 | OH |
| 128 | Pr | (III) | NHCO | 4-indanyl | 3 | $NH_2$ |
| 129 | Pr | (II) | $NHCH_2$ | 4-indanyl | 0 | - |
| 130 | Pr | (II) | $NHCH_2$ | 4-indanyl | 1 | - |
| 131 | Pr | (III) | $NHCH_2$ | 4-indanyl | 1 | OH |
| 132 | Pr | (III) | $NHCH_2$ | 4-indanyl | 1 | $NH_2$ |
| 133 | Pr | (III) | $NHCH_2$ | 4-indanyl | 2 | OH |
| 134 | Pr | (III) | $NHCH_2$ | 4-indanyl | 2 | $NH_2$ |
| 135 | Pr | (III) | $NHCH_2$ | 4-indanyl | 3 | OH |
| 136 | Pr | (III) | $NHCH_2$ | 4-indanyl | 3 | $NH_2$ |
| 137 | Pr | (II) | CONH | 4-indanyl | 0 | - |
| 138 | Pr | (II) | CONH | 4-indanyl | 1 | - |
| 139 | Pr | (II) | $CH_2NH$ | 4-indanyl | 0 | - |
| 140 | Pr | (II) | $CH_2NH$ | 4-indanyl | 1 | - |
| 141 | Pr | (II) | CO | 4-indanyl | 0 | - |
| 122 | Pr | (II) | NHCO | 4-indanyl | 0 | - |

Table 5 (continued)

| Compound | $R^1$ | $R^2$ | X | Ar | m or n | Y |
|---|---|---|---|---|---|---|
| 143 | Pr | (III) | NHCO | 4-indanyl | 1 | OH |
| 144 | Pr | (III) | NHCO | 4-indanyl | 1 | $NH_2$ |
| 145 | Pr | (II) | NHCO | 4-indanyl | 0 | - |
| 146 | Pr | (III) | NHCO | 4-indanyl | 1 | $NH_2$ |
| 147 | Pr | (II) | NHCO | 4-indanyl | 0 | - |
| 148 | Pr | (III) | NHCO | 4-indanyl | 1 | $NH_2$ |
| 149 | Pr | (II) | NHCO | 4-indanyl | 0 | - |
| 150 | Pr | (III) | NHCO | 4-indanyl | 1 | $NH_2$ |

Table 6

| Compound | $R^1$ | $R^2$ | X | Ar | m or n | Y |
|---|---|---|---|---|---|---|
| 151 | H | (II) | NHCO | 5,6,7,8-tetrahydro-1-naphthyl | 0 | - |
| 152 | H | (II) | NHCO | 5,6,7,8-tetrahydro-1-naphthyl | 1 | - |
| 153 | H | (III) | NHCO | 5,6,7,8-tetrahydro-1-naphthyl | 1 | OH |
| 154 | H | (III) | NHCO | 5,6,7,8-tetrahydro-1-naphthyl | 1 | $NH_2$ |
| 155 | H | (III) | NHCO | 5,6,7,8-tetrahydro-1-naphthyl | 2 | OH |
| 156 | H | (III) | NHCO | 5,6,7,8-tetrahydro-1-naphthyl | 2 | $NH_2$ |
| 157 | H | (III) | NHCO | 5,6,7,8-tetrahydro-1-naphthyl | 3 | OH |
| 158 | H | (III) | NHCO | 5,6,7,8-tetrahydro-1-naphthyl | 3 | $NH_2$ |
| 159 | H | (II) | $NHCH_2$ | 5,6,7,8-tetrahydro-1-naphthyl | 0 | - |
| 160 | H | (II) | $NHCH_2$ | 5,6,7,8-tetrahydro-1-naphthyl | 1 | - |
| 161 | H | (III) | $NHCH_2$ | 5,6,7,8-tetrahydro-1-naphthyl | 1 | OH |
| 162 | H | (III) | $NHCH_2$ | 5,6,7,8-tetrahydro-1-naphthyl | 1 | $NH_2$ |
| 163 | H | (III) | $NHCH_2$ | 5,6,7,8-tetrahydro-1-naphthyl | 2 | OH |
| 164 | H | (III) | $NHCH_2$ | 5,6,7,8-tetrahydro-1-naphthyl | 2 | $NH_2$ |
| 165 | H | (III) | $NHCH_2$ | 5,6,7,8-tetrahydro-1-naphthyl | 3 | OH |
| 166 | H | (III) | $NHCH_2$ | 5,6,7,8-tetrahydro-1-naphthyl | 3 | $NH_2$ |
| 167 | H | (II) | CONH | 5,6,7,8-tetrahydro-1-naphthyl | 0 | - |
| 168 | H | (II) | CONH | 5,6,7,8-tetrahydro-1-naphthyl | 1 | - |
| 169 | H | (II) | $CH_2NH$ | 5,6,7,8-tetrahydro-1-naphthyl | 0 | - |
| 170 | H | (II) | $CH_2NH$ | 5,6,7,8-tetrahydro-1-naphthyl | 1 | - |
| 171 | H | (II) | CO | 5,6,7,8-tetrahydro-1-naphthyl | 0 | - |
| 172 | H | (II) | NHCO | 5,6,7,8-tetrahydro-1-naphthyl | 0 | - |
| 173 | H | (III) | NHCO | 5,6,7,8-tetrahydro-1-naphthyl | 1 | OH |
| 174 | H | (III) | NHCO | 5,6,7,8-tetrahydro-1-naphthyl | 1 | $NH_2$ |
| 175 | H | (II) | NHCO | 5,6,7,8-tetrahydro-1-naphthyl | 0 | - |
| 176 | H | (III) | NHCO | 5,6,7,8-tetrahydro-1-naphthyl | 1 | $NH_2$ |
| 177 | H | (II) | NHCO | 5,6,7,8-tetrahydro-1-naphthyl | 0 | - |

Table 6 (continued)

| Compound | $R^1$ | $R^2$ | X | Ar | m or n | Y |
|---|---|---|---|---|---|---|
| 178 | H | (III) | NHCO | 5,6,7,8-tetrahydro-1-naphthyl | 1 | $NH_2$ |
| 179 | H | (II) | NHCO | 5,6,7,8-tetrahydro-1-naphthyl | 0 | - |
| 180 | H | (III) | NHCO | 5,6,7,8-tetrahydro-1-naphthyl | 1 | $NH_2$ |

Table 7

| Compound | $R^1$ | $R^2$ | X | Ar | m or n | Y |
|---|---|---|---|---|---|---|
| 181 | Me | (II) | NHCO | 5,6,7,8-tetrahydro-1-naphthyl | 0 | - |
| 182 | Me | (II) | NHCO | 5,6,7,8-tetrahydro-1-naphthyl | 1 | - |
| 183 | Me | (III) | NHCO | 5,6,7,8-tetrahydro-1-naphthyl | 1 | OH |
| 184 | Me | (III) | NHCO | 5,6,7,8-tetrahydro-1-naphthyl | 1 | $NH_2$ |
| 185 | Me | (III) | NHCO | 5,6,7,8-tetrahydro-1-naphthyl | 2 | OH |
| 186 | Me | (III) | NHCO | 5,6,7,8-tetrahydro-1-naphthyl | 2 | $NH_2$ |
| 187 | Me | (III) | NHCO | 5,6,7,8-tetrahydro-1-naphthyl | 3 | OH |
| 188 | Me | (III) | NHCO | 5,6,7,8-tetrahydro-1-naphthyl | 3 | $NH_2$ |
| 189 | Me | (II) | $NHCH_2$ | 5,6,7,8-tetrahydro-1-naphthyl | 0 | - |
| 190 | Me | (II) | $NHCH_2$ | 5,6,7,8-tetrahydro-1-naphthyl | 1 | - |
| 191 | Me | (III) | $NHCH_2$ | 5,6,7,8-tetrahydro-1-naphthyl | 1 | OH |
| 192 | Me | (III) | $NHCH_2$ | 5,6,7,8-tetrahydro-1-naphthyl | 1 | $NH_2$ |
| 193 | Me | (III) | $NHCH_2$ | 5,6,7,8-tetrahydro-1-naphthyl | 2 | OH |
| 194 | Me | (III) | $NHCH_2$ | 5,6,7,8-tetrahydro-1-naphthyl | 2 | $NH_2$ |
| 195 | Me | (III) | $NHCH_2$ | 5,6,7,8-tetrahydro-1-naphthyl | 3 | OH |
| 196 | Me | (III) | $NHCH_2$ | 5,6,7,8-tetrahydro-1-naphthyl | 3 | $NH_2$ |
| 197 | Me | (II) | CONH | 5,6,7,8-tetrahydro-1-naphthyl | 0 | - |
| 198 | Me | (II) | CONH | 5,6,7,8-tetrahydro-1-naphthyl | 1 | - |
| 199 | Me | (II) | $CH_2NH$ | 5,6,7,8-tetrahydro-1-naphthyl | 0 | - |
| 200 | Me | (II) | $CH_2NH$ | 5,6,7,8-tetrahydro-1-naphthyl | 1 | - |
| 201 | Me | (II) | CO | 5,6,7,8-tetrahydro-1-naphthyl | 0 | - |
| 202 | Me | (II) | NHCO | 5,6,7,8-tetrahydro-1-naphthyl | 0 | - |
| 203 | Me | (III) | NHCO | 5,6,7,8-tetrahydro-1-naphthyl | 1 | OH |
| 204 | Me | (III) | NHCO | 5,6,7,8-tetrahydro-1-naphthyl | 1 | $NH_2$ |
| 205 | Me | (II) | NHCO | 5,6,7,8-tetrahydro-1-naphthyl | 0 | - |
| 206 | Me | (III) | NHCO | 5,6,7,8-tetrahydro-1-naphthyl | 1 | $NH_2$ |
| 207 | Me | (II) | NHCO | 5,6,7,8-tetrahydro-1-naphthyl | 0 | - |
| 208 | Me | (III) | NHCO | 5,6,7,8-tetrahydro-1-naphthyl | 1 | $NH_2$ |
| 209 | Me | (II) | NHCO | 5,6,7,8-tetrahydro-1-naphthyl | 0 | - |
| 210 | Me | (III) | NHCO | 5,6,7,8-tetrahydro-1-naphthyl | 1 | $NH_2$ |

Table 8

| Compound | $R^1$ | $R^2$ | X | Ar | m or n | Y |
|---|---|---|---|---|---|---|
| 211 | Et | (II) | NHCO | 5,6,7,8-tetrahydro-1-naphthyl | 0 | - |
| 212 | Et | (II) | NHCO | 5,6,7,8-tetrahydro-1-naphthyl | 1 | - |
| 213 | Et | (III) | NHCO | 5,6,7,8-tetrahydro-1-naphthyl | 1 | OH |
| 214 | Et | (III) | NHCO | 5,6,7,8-tetrahydro-1-naphthyl | 1 | $NH_2$ |
| 215 | Et | (III) | NHCO | 5,6,7,8-tetrahydro-1-naphthyl | 2 | OH |
| 216 | Et | (III) | NHCO | 5,6,7,8-tetrahydro-1-naphthyl | 2 | $NH_2$ |
| 217 | Et | (III) | NHCO | 5,6,7,8-tetrahydro-1-naphthyl | 3 | OH |
| 218 | Et | (III) | NHCO | 5,6,7,8-tetrahydro-1-naphthyl | 3 | $NH_2$ |
| 219 | Et | (II) | $NHCH_2$ | 5,6,7,8-tetrahydro-1-naphthyl | 0 | - |
| 220 | Et | (II) | $NHCH_2$ | 5,6,7,8-tetrahydro-1-naphthyl | 1 | - |
| 221 | Et | (III) | $NHCH_2$ | 5,6,7,8-tetrahydro-1-naphthyl | 1 | OH |
| 222 | Et | (III) | $NHCH_2$ | 5,6,7,8-tetrahydro-1-naphthyl | 1 | $NH_2$ |
| 223 | Et | (III) | $NHCH_2$ | 5,6,7,8-tetrahydro-1-naphthyl | 2 | OH |
| 224 | Et | (III) | $NHCH_2$ | 5,6,7,8-tetrahydro-1-naphthyl | 2 | $NH_2$ |
| 225 | Et | (III) | $NHCH_2$ | 5,6,7,8-tetrahydro-1-naphthyl | 3 | OH |
| 226 | Et | (III) | $NHCH_2$ | 5,6,7,8-tetrahydro-1-naphthyl | 3 | $NH_2$ |
| 227 | Et | (II) | CONH | 5,6,7,8-tetrahydro-1-naphthyl | 0 | - |
| 228 | Et | (II) | CONH | 5,6,7,8-tetrahydro-1-naphthyl | 1 | - |
| 229 | Et | (II) | $CH_2NH$ | 5,6,7,8-tetrahydro-1-naphthyl | 0 | - |
| 230 | Et | (II) | $CH_2NH$ | 5,6,7,8-tetrahydro-1-naphthyl | 1 | - |
| 231 | Et | (II) | CO | 5,6,7,8-tetrahydro-1-naphthyl | 0 | - |
| 232 | Et | (II) | NHCO | 5,6,7,8-tetrahydro-1-naphthyl | 0 | - |
| 233 | Et | (III) | NHCO | 5,6,7,8-tetrahydro-1-naphthyl | 1 | OH |
| 234 | Et | (III) | NHCO | 5,6,7,8-tetrahydro-1-naphthyl | 1 | $NH_2$ |
| 235 | Et | (II) | NHCO | 5,6,7,8-tetrahydro-1-naphthyl | 0 | - |
| 236 | Et | (III) | NHCO | 5,6,7,8-tetrahydro-1-naphthyl | 1 | $NH_2$ |
| 237 | Et | (II) | NHCO | 5,6,7,8-tetrahydro-1-naphthyl | 0 | - |
| 238 | Et | (III) | NHCO | 5,6,7,8-tetrahydro-1-naphthyl | 1 | $NH_2$ |
| 239 | Et | (II) | NHCO | 5,6,7,8-tetrahydro-1-naphthyl | 0 | - |
| 240 | Et | (III) | NHCO | 5,6,7,8-tetrahydro-1-naphthyl | 1 | $NH_2$ |

Table 9

| Compound | $R^1$ | $R^2$ | X | Ar | m or n | Y |
|---|---|---|---|---|---|---|
| 241 | Pr | (II) | NHCO | 5,6,7,8-tetrahydro-1-naphthyl | 0 | - |
| 242 | Pr | (II) | NHCO | 5,6,7,8-tetrahydro-1-naphthyl | 1 | - |
| 243 | Pr | (III) | NHCO | 5,6,7,8-tetrahydro-1-naphthyl | 1 | OH |
| 244 | Pr | (III) | NHCO | 5,6,7,8-tetrahydro-1-naphthyl | 1 | $NH_2$ |

Table 9 (continued)

| Compound | $R^1$ | $R^2$ | X | Ar | m or n | Y |
|---|---|---|---|---|---|---|
| 245 | Pr | (III) | NHCO | 5,6,7,8-tetrahydro-1-naphthyl | 2 | OH |
| 246 | Pr | (III) | NHCO | 5,6,7,8-tetrahydro-1-naphthyl | 2 | $NH_2$ |
| 247 | Pr | (III) | NHCO | 5,6,7,8-tetrahydro-1-naphthyl | 3 | OH |
| 248 | Pr | (III) | NHCO | 5,6,7,8-tetrahydro-1-naphthyl | 3 | $NH_2$ |
| 249 | Pr | (II) | $NHCH_2$ | 5,6,7,8-tetrahydro-1-naphthyl | 0 | - |
| 250 | Pr | (II) | $NHCH_2$ | 5,6,7,8-tetrahydro-1-naphthyl | 1 | - |
| 251 | Pr | (III) | $NHCH_2$ | 5,6,7,8-tetrahydro-1-naphthyl | 1 | OH |
| 252 | Pr | (III) | $NHCH_2$ | 5,6,7,8-tetrahydro-1-naphthyl | 1 | $NH_2$ |
| 253 | Pr | (III) | $NHCH_2$ | 5,6,7,8-tetrahydro-1-naphthyl | 2 | OH |
| 254 | Pr | (III) | $NHCH_2$ | 5,6,7,8-tetrahydro-1-naphthyl | 2 | $NH_2$ |
| 255 | Pr | (III) | $NHCH_2$ | 5,6,7,8-tetrahydro-1-naphthyl | 3 | OH |
| 256 | Pr | (III) | $NHCH_2$ | 5,6,7,8-tetrahydro-1-naphthyl | 3 | $NH_2$ |
| 257 | Pr | (II) | CONH | 5,6,7,8-tetrahydro-1-naphthyl | 0 | - |
| 258 | Pr | (II) | CONH | 5,6,7,8-tetrahydro-1-naphthyl | 1 | - |
| 259 | Pr | (II) | $CH_2NH$ | 5,6,7,8-tetrahydro-1-naphthyl | 0 | - |
| 260 | Pr | (II) | $CH_2NH$ | 5,6,7,8-tetrahydro-1-naphthyl | 1 | - |
| 261 | Pr | (II) | CO | 5,6,7,8-tetrahydro-1-naphthyl | 0 | - |
| 262 | Pr | (II) | NHCO | 5,6,7,8-tetrahydro-1-naphthyl | 0 | - |
| 263 | Pr | (III) | NHCO | 5,6,7,8-tetrahydro-1-naphthyl | 1 | OH |
| 264 | Pr | (III) | NHCO | 5,6,7,8-tetrahydro-1-naphthyl | 1 | $NH_2$ |
| 265 | Pr | (II) | NHCO | 5,6,7,8-tetrahydro-1-naphthyl | 0 | - |
| 266 | Pr | (III) | NHCO | 5,6,7,8-tetrahydro-1-naphthyl | 1 | $NH_2$ |
| 267 | Pr | (II) | NHCO | 5,6,7,8-tetrahydro-1-naphthyl | 0 | - |
| 268 | Pr | (III) | NHCO | 5,6,7,8-tetrahydro-1-naphthyl | 1 | $NH_2$ |
| 269 | Pr | (II) | NHCO | 5,6,7,8-tetrahydro-1-naphthyl | 0 | - |
| 270 | Pr | (III) | NHCO | 5,6,7,8-tetrahydro-1-naphthyl | 1 | $NH_2$ |

Table 10

| Compound | $R^1$ | $R^2$ | X | Ar | m or n | Y |
|---|---|---|---|---|---|---|
| 271 | H | (II) | NHCO | 2,3-dimethylphenyl | 0 | - |
| 272 | H | (II) | NHCO | 2,3-dimethylphenyl | 1 | - |
| 273 | H | (III) | NHCO | 2,3-dimethylphenyl | 1 | OH |
| 274 | H | (III) | NHCO | 2,3- dimethylphenyl | 1 | $NH_2$ |
| 275 | H | (III) | NHCO | 2,3-dimethylphenyl | 2 | OH |
| 276 | H | (III) | NHCO | 2,3-dimethylphenyl | 2 | $NH_2$ |
| 277 | H | (III) | NHCO | 2,3-dimethylphenyl | 3 | OH |
| 278 | H | (III) | NHCO | 2,3- dimethylphenyl | 3 | $NH_2$ |
| 279 | H | (II) | $NHCH_2$ | 2,3-dimethylphenyl | 0 | - |

Table 10 (continued)

| Compound | $R^1$ | $R^2$ | X | Ar | m or n | Y |
|---|---|---|---|---|---|---|
| 280 | H | (II) | $NHCH_2$ | 2,3-dimethylphenyl | 1 | - |
| 281 | H | (III) | $NHCH_2$ | 2,3-dimethylphenyl | 1 | OH |
| 282 | H | (III) | $NHCH_2$ | 2,3-dimethylphenyl | 1 | $NH_2$ |
| 283 | H | (III) | $NHCH_2$ | 2,3-dimethylphenyl | 2 | OH |
| 284 | H | (III) | $NHCH_2$ | 2,3-dimethylphenyl | 2 | $NH_2$ |
| 285 | H | (III) | $NHCH_2$ | 2,3-dimethylphenyl | 3 | OH |
| 286 | H | (III) | $NHCH_2$ | 2,3-dimethylphenyl | 3 | $NH_2$ |
| 287 | H | (II) | CONH | 2,3-dimethylphenyl | 0 | - |
| 288 | H | (II) | CONH | 2,3-dimethylphenyl | 1 | - |
| 289 | H | (II) | $CH_2NH$ | 2,3-dimethylphenyl | 0 | - |
| 290 | H | (II) | $CH_2NH$ | 2,3-dimethylphenyl | 1 | - |
| 291 | H | (II) | CO | 2,3-dimethylphenyl | 0 | - |
| 292 | H | (II) | NHCO | 2,3-dimethylphenyl | 0 | - |
| 293 | H | (III) | NHCO | 2,3-dimethylphenyl | 1 | OH |
| 294 | H | (III) | NHCO | 2,3-dimethylphenyl | 1 | $NH_2$ |
| 295 | H | (II) | NHCO | 2,3-dimethylphenyl | 0 | - |
| 296 | H | (III) | NHCO | 2,3-dimethylphenyl | 1 | $NH_2$ |
| 297 | H | (II) | NHCO | 2,3-dimethylphenyl | 0 | - |
| 298 | H | (III) | NHCO | 2,3-dimethylphenyl | 1 | $NH_2$ |
| 299 | H | (II) | NHCO | 2,3-dimethylphenyl | 0 | - |
| 300 | H | (III) | NHCO | 2,3-dimethylphenyl | 1 | $NH_2$ |

Table 11

| Compound | $R^1$ | $R^2$ | X | Ar | m or n | Y |
|---|---|---|---|---|---|---|
| 301 | Me | (II) | NHCO | 2,3-dimethylphenyl | 0 | - |
| 302 | Me | (II) | NHCO | 2,3-dimethylphenyl | 1 | - |
| 303 | Me | (III) | NHCO | 2,3-dimethylphenyl | 1 | OH |
| 304 | Me | (III) | NHCO | 2,3-dimethylphenyl | 1 | $NH_2$ |
| 305 | Me | (III) | NHCO | 2,3-dimethylphenyl | 2 | OH |
| 306 | Me | (III) | NHCO | 2,3-dimethylphenyl | 2 | $NH_2$ |
| 307 | Me | (III) | NHCO | 2,3-dimethylphenyl | 3 | OH |
| 308 | Me | (III) | NHCO | 2,3-dimethylphenyl | 3 | $NH_2$ |
| 309 | Me | (II) | $NHCH_2$ | 2,3-dimethylphenyl | 0 | - |
| 310 | Me | (II) | $NHCH_2$ | 2,3-dimethylphenyl | 1 | - |
| 311 | Me | (III) | $NHCH_2$ | 2,3-dimethylphenyl | 1 | OH |
| 312 | Me | (III) | $NHCH_2$ | 2,3-dimethylphenyl | 1 | $NH_2$ |
| 313 | Me | (III) | $NHCH_2$ | 2,3-dimethylphenyl | 2 | OH |
| 314 | Me | (III) | $NHCH_2$ | 2,3-dimethylphenyl | 2 | $NH_2$ |

Table 11 (continued)

| Compound | $R^1$ | $R^2$ | X | Ar | m or n | Y |
|---|---|---|---|---|---|---|
| 315 | Me | (III) | $NHCH_2$ | 2,3-dimethylphenyl | 3 | OH |
| 316 | Me | (III) | $NHCH_2$ | 2,3-dimethylphenyl | 3 | $NH_2$ |
| 317 | Me | (II) | CONH | 2,3-dimethylphenyl | 0 | - |
| 318 | Me | (II) | CONH | 2,3-dimethylphenyl | 1 | - |
| 319 | Me | (II) | $CH_2NH$ | 2,3-dimethylphenyl | 0 | - |
| 320 | Me | (II) | $CH_2NH$ | 2,3-dimethylphenyl | 1 | - |
| 321 | Me | (II) | CO | 2,3-dimethylphenyl | 0 | - |
| 322 | Me | (II) | NHCO | 2,3-dimethylphenyl | 0 | - |
| 323 | Me | (III) | NHCO | 2,3-dimethylphenyl | 1 | OH |
| 324 | Me | (III) | NHCO | 2,3-dimethylphenyl | 1 | $NH_2$ |
| 325 | Me | (II) | NHCO | 2,3-dimethylphenyl | 0 | - |
| 326 | Me | (III) | NHCO | 2,3-dimethylphenyl | 1 | $NH_2$ |
| 327 | Me | (II) | NHCO | 2,3-dimethylphenyl | 0 | - |
| 328 | Me | (III) | NHCO | 2,3-dimethylphenyl | 1 | $NH_2$ |
| 329 | Me | (II) | NHCO | 2,3-dimethylphenyl | 0 | - |
| 330 | Me | (III) | NHCO | 2,3-dimethylphenyl | 1 | $NH_2$ |

Table 12

| Compound | $R^1$ | $R^2$ | X | Ar | m or n | Y |
|---|---|---|---|---|---|---|
| 331 | Et | (II) | NHCO | 2,3-dimethylphenyl | 0 | - |
| 332 | Et | (II) | NHCO | 2,3-dimethylphenyl | 1 | - |
| 333 | Et | (III) | NHCO | 2,3-dimethylphenyl | 1 | OH |
| 334 | Et | (III) | NHCO | 2,3-dimethylphenyl | 1 | $NH_2$ |
| 335 | Et | (III) | NHCO | 2,3-dimethylphenyl | 2 | OH |
| 336 | Et | (III) | NHCO | 2,3-dimethylphenyl | 2 | $NH_2$ |
| 337 | Et | (III) | NHCO | 2,3-dimethylphenyl | 3 | OH |
| 338 | Et | (III) | NHCO | 2,3-dimethylphenyl | 3 | $NH_2$ |
| 339 | Et | (II) | $NHCH_2$ | 2,3-dimethylphenyl | 0 | - |
| 340 | Et | (II) | $NHCH_2$ | 2,3-dimethylphenyl | 1 | - |
| 341 | Et | (III) | $NHCH_2$ | 2,3-dimethylphenyl | 1 | OH |
| 342 | Et | (III) | $NHCH_2$ | 2,3-dimethylphenyl | 1 | $NH_2$ |
| 343 | Et | (III) | $NHCH_2$ | 2,3-dimethylphenyl | 2 | OH |
| 344 | Et | (III) | $NHCH_2$ | 2,3-dimethylphenyl | 2 | $NH_2$ |
| 345 | Et | (III) | $NHCH_2$ | 2,3-dimethylphenyl | 3 | OH |
| 346 | Et | (III) | $NHCH_2$ | 2,3-dimethylphenyl | 3 | $NH_2$ |
| 347 | Et | (II) | CONH | 2,3-dimethylphenyl | 0 | - |
| 348 | Et | (II) | CONH | 2,3-dimethylphenyl | 1 | - |
| 349 | Et | (II) | $CH_2NH$ | 2,3-dimethylphenyl | 0 | - |

Table 12 (continued)

| Compound | $R^1$ | $R^2$ | X | Ar | m or n | Y |
|---|---|---|---|---|---|---|
| 350 | Et | (II) | $CH_2NH$ | 2,3-dimethylphenyl | 1 | - |
| 351 | Et | (II) | CO | 2,3-dimethylphenyl | 0 | - |
| 352 | Et | (II) | NHCO | 2,3-dimethylphenyl | 0 | - |
| 353 | Et | (III) | NHCO | 2,3-dimethylphenyl | 1 | OH |
| 354 | Et | (III) | NHCO | 2,3-dimethylphenyl | 1 | $NH_2$ |
| 355 | Et | (II) | NHCO | 2,3-dimethylphenyl | 0 | - |
| 356 | Et | (III) | NHCO | 2,3-dimethylphenyl | 1 | $NH_2$ |
| 357 | Et | (II) | NHCO | 2,3-dimethylphenyl | 0 | - |
| 358 | Et | (III) | NHCO | 2,3-dimethylphenyl | 1 | $NH_2$ |
| 359 | Et | (II) | NHCO | 2,3-dimethylphenyl | 0 | - |
| 360 | Et | (III) | NHCO | 2,3-dimethylphenyl | 1 | $NH_2$ |

Table 13

| Compound | $R^1$ | $R^2$ | X | Ar | m or n | Y |
|---|---|---|---|---|---|---|
| 361 | Pr | (II) | NHCO | 2,3-dimethylphenyl | 0 | - |
| 362 | Pr | (II) | NHCO | 2,3-dimethylphenyl | 1 | - |
| 363 | Pr | (III) | NHCO | 2,3-dimethylphenyl | 1 | OH |
| 364 | Pr | (III) | NHCO | 2,3-dimethylphenyl | 1 | $NH_2$ |
| 365 | Pr | (III) | NHCO | 2,3-dimethylphenyl | 2 | OH |
| 366 | Pr | (III) | NHCO | 2,3-dimethylphenyl | 2 | $NH_2$ |
| 367 | Pr | (III) | NHCO | 2,3-dimethylphenyl | 3 | OH |
| 368 | Pr | (III) | NHCO | 2,3-dimethylphenyl | 3 | $NH_2$ |
| 369 | Pr | (II) | $NHCH_2$ | 2,3-dimethylphenyl | 0 | - |
| 370 | Pr | (II) | $NHCH_2$ | 2,3-dimethylphenyl | 1 | - |
| 371 | Pr | (III) | $NHCH_2$ | 2,3-dimethylphenyl | 1 | OH |
| 372 | Pr | (III) | $NHCH_2$ | 2,3-dimethylphenyl | 1 | $NH_2$ |
| 373 | Pr | (III) | $NHCH_2$ | 2,3-dimethylphenyl | 2 | OH |
| 374 | Pr | (III) | $NHCH_2$ | 2,3-dimethylphenyl | 2 | $NH_2$ |
| 375 | Pr | (III) | $NHCH_2$ | 2,3-dimethylphenyl | 3 | OH |
| 376 | Pr | (III) | $NHCH_2$ | 2,3-dimethylphenyl | 3 | $NH_2$ |
| 377 | Pr | (II) | CONH | 2,3-dimethylphenyl | 0 | - |
| 378 | Pr | (II) | CONH | 2,3-dimethylphenyl | 1 | - |
| 379 | Pr | (II) | $CH_2NH$ | 2,3-dimethylphenyl | 0 | - |
| 380 | Pr | (II) | $CH_2NH$ | 2,3-dimethylphenyl | 1 | - |
| 381 | Pr | (II) | CO | 2,3-dimethylphenyl | 0 | - |
| 382 | Pr | (II) | NHCO | 2,3-dimethylphenyl | 0 | - |
| 383 | Pr | (III) | NHCO | 2,3-dimethylphenyl | 1 | OH |
| 384 | Pr | (III) | NHCO | 2,3-dimethylphenyl | 1 | $NH_2$ |

Table 13 (continued)

| Compound | $R^1$ | $R^2$ | X | Ar | m or n | Y |
|---|---|---|---|---|---|---|
| 385 | Pr | (II) | NHCO | 2,3-dimethylphenyl | 0 | - |
| 386 | Pr | (III) | NHCO | 2,3-dimethylphenyl | 1 | $NH_2$ |
| 387 | Pr | (II) | NHCO | 2,3-dimethylphenyl | 0 | - |
| 388 | Pr | (III) | NHCO | 2,3-dimethylphenyl | 1 | $NH_2$ |
| 389 | Pr | (II) | NHCO | 2,3-dimethylphenyl | 0 | - |
| 390 | Pr | (III) | NHCO | 2,3-dimethylphenyl | 1 | $NH_2$ |

Table 14

| Compound | $R^1$ | $R^2$ | X | Ar | m or n | Y |
|---|---|---|---|---|---|---|
| 391 | Me | (II) | NHCO | 3-Py | 0 | - |
| 392 | Me | (II) | NHCO | 3-Py | 1 | - |
| 393 | Me | (III) | NHCO | 3-Py | 1 | OH |
| 394 | Me | (III) | NHCO | 3-Py | 1 | $NH_2$ |
| 395 | Me | (III) | NHCO | 3-Py | 2 | OH |
| 396 | Me | (III) | NHCO | 3-Py | 2 | $NH_2$ |
| 397 | Me | (III) | NHCO | 3-Py | 3 | OH |
| 398 | Me | (III) | NHCO | 3-Py | 3 | $NH_2$ |
| 399 | Et | (II) | NHCO | 3-Py | 0 | - |
| 400 | Et | (II) | NHCO | 3-Py | 1 | - |
| 401 | Et | (III) | NHCO | 3-Py | 1 | OH |
| 402 | Et | (III) | NHCO | 3-Py | 1 | $NH_2$ |
| 403 | Et | (III) | NHCO | 3-Py | 2 | OH |
| 404 | Et | (III) | NHCO | 3-Py | 2 | $NH_2$ |
| 405 | Et | (III) | NHCO | 3-Py | 3 | OH |
| 406 | Et | (III) | NHCO | 3-Py | 3 | $NH_2$ |
| 407 | Pr | (II) | NHCO | 3-Py | 0 | - |
| 408 | Pr | (II) | NHCO | 3-Py | 1 | - |
| 409 | Pr | (III) | NHCO | 3-Py | 1 | OH |
| 410 | Pr | (III) | NHCO | 3-Py | 0 | $NH_2$ |
| 411 | Pr | (III) | NHCO | 3-Py | 1 | OH |
| 412 | Pr | (III) | NHCO | 3-Py | 0 | $NH_2$ |
| 413 | Pr | (III) | NHCO | 3-Py | 1 | $NH_2$ |
| 414 | Pr | (III) | NHCO | 3-Py | 0 | OH |
| 415 | Pr | (III) | NHCO | 3-Py | 1 | $NH_2$ |

Table 15

| Compound | $R^1$ | $R^2$ | X | Ar | m or n | Y |
|---|---|---|---|---|---|---|
| 416 | Me | (II) | NHCO | 2-Py | 0 | - |

Table 15 (continued)

| Compound | $R^1$ | $R^2$ | X | Ar | m or n | Y |
|---|---|---|---|---|---|---|
| 417 | Me | (II) | NHCO | 2-Py | 1 | - |
| 418 | Et | (III) | NHCO | 2-Py | 0 | OH |
| 419 | Et | (III) | NHCO | 2-Py | 1 | $NH_2$ |
| 420 | Pr | (III) | NHCO | 2-Py | 0 | OH |
| 421 | Pr | (III) | NHCO | 2-Py | 1 | $NH_2$ |
| 422 | Me | (III) | NHCO | 2-Me-3-Py | 0 | OH |
| 423 | Me | (III) | NHCO | 2-Me-3-Py | 1 | $NH_2$ |
| 424 | Et | (II) | NHCO | 2-Me-3-Py | 0 | - |
| 425 | Et | (II) | NHCO | 2-Me-3-Py | 1 | - |
| 426 | Pr | (III) | NHCO | 2-Me-3-Py | 0 | OH |
| 427 | Pr | (III) | NHCO | 2-Me-3-Py | 1 | $NH_2$ |
| 428 | Me | (III) | NHCO | 2-OMe-3-Py | 0 | OH |
| 429 | Me | (III) | NHCO | 2-OMe-3-Py | 1 | $NH_2$ |
| 430 | Et | (III) | NHCO | 2-OMe-3-Py | 0 | OH |
| 431 | Et | (III) | NHCO | 2-OMe-3-Py | 1 | $NH_2$ |
| 432 | Pr | (II) | NHCO | 2-OMe-3-Py | 0 | - |
| 433 | Pr | (II) | NHCO | 2-OMe-3-Py | 1 | - |
| 434 | Me | (III) | NHCO | 6-OMe-Py | 0 | OH |
| 435 | Me | (III) | NHCO | 6-OMe-Py | 1 | $NH_2$ |
| 436 | Et | (III) | NHCO | 6-OMe-Py | 0 | OH |
| 437 | Et | (III) | NHCO | 6-OMe-Py | 1 | $NH_2$ |
| 438 | Et | (III) | NHCO | 6-OMe-Py | 0 | - |
| 439 | Et | (III) | NHCO | 6-OMe-Py | 1 | - |

Table 16

| Compound | $R^1$ | $R^2$ | X | Ar | m or n | Y |
|---|---|---|---|---|---|---|
| 440 | Me | (II) | NHCO | 4-Py | 0 | - |
| 441 | Me | (II) | NHCO | 4-Py | 1 | - |
| 442 | Et | (III) | NHCO | 4-Py | 0 | OH |
| 443 | Et | (III) | NHCO | 4-Py | 1 | $NH_2$ |
| 444 | Pr | (III) | NHCO | 4-Py | 0 | OH |
| 445 | Pr | (III) | NHCO | 4-Py | 1 | $NH_2$ |
| 446 | Me | (III) | NHCO | 2,6-OMe-3-Py | 0 | OH |
| 447 | Me | (III) | NHCO | 2,6-OMe-3-Py | 1 | $NH_2$ |
| 448 | Et | (II) | NHCO | 2,6-OMe-3-Py | 0 | - |
| 449 | Et | (II) | NHCO | 2,6-OMe-3-Py | 1 | - |
| 450 | Et | (III) | NHCO | 2,6-OMe-3-Py | 0 | OH |
| 451 | Et | (III) | NHCO | 2,6-OMe-3-Py | 1 | $NH_2$ |

Table 16 (continued)

| Compound | $R^1$ | $R^2$ | X | Ar | m or n | Y |
|---|---|---|---|---|---|---|
| 452 | Me | (III) | NHCO | 6-Cl-3-Py | 0 | OH |
| 453 | Me | (III) | NHCO | 6-Cl-3-Py | 1 | $NH_2$ |
| 454 | Et | (III) | NHCO | 6-Cl-3-Py | 0 | OH |
| 455 | Et | (III) | NHCO | 6-Cl-3-Py | 1 | $NH_2$ |
| 456 | Et | (II) | NHCO | 6-Cl-3-Py | 0 | - |
| 457 | Et | (II) | NHCO | 6-Cl-3-Py | 1 | - |
| 458 | Me | (III) | NHCO | 2,3-diethylphe nyl | 0 | OH |
| 459 | Me | (III) | NHCO | 2,3-diethylphe nyl | 1 | $NH_2$ |
| 460 | Et | (III) | NHCO | 2,3-diethylphe nyl | 0 | OH |
| 461 | Et | (III) | NHCO | 2,3-diethylphe nyl | 1 | $NH_2$ |
| 462 | Pr | (III) | NHCO | 2,3-diethylphe nyl | 0 | OH |
| 463 | Pr | (III) | NHCO | 2,3-diethylphe nyl | 1 | $NH_2$ |

Table 17

| Compound | $R^1$ | $R^2$ | X | Ar | m or n | Y |
|---|---|---|---|---|---|---|
| 464 | (CH2)2OMe | (II) | NHCO | 2,3-Me-Ph | 0 | - |
| 465 | (CH2)3OMe | (II) | NHCO | 2,3-Me-Ph | 0 | - |
| 466 | (CH2)2OH | (II) | NHCO | 2,3-Me-Ph | 0 | - |
| 467 | (CH2)3OH | (II) | NHCO | 2,3-Me-Ph | 0 | - |
| 468 | (CH2)40H | (II) | NHCO | 2,3-Me-Ph | 0 | - |
| 469 | (CH2)2OH | (II) | NHCO | 2,3-Me-Ph | 1 | - |
| 470 | (CH2)3OH | (II) | NHCO | 2,3-Me-Ph | 1 | - |
| 471 | (CH2)4OH | (II) | NHCO | 2,3-Me-Ph | 1 | - |
| 472 | (CH2)2OH | (III) | NHCO | 2,3-Me-Ph | 1 | $NH_2$ |
| 473 | (CH2)3OH | (III) | NHCO | 2,3-Me-Ph | 1 | $NH_2$ |
| 474 | (CH2)4OH | (III) | NHCO | 2,3-Me-Ph | 1 | $NH_2$ |
| 475 | (CH2)2OH | (II) | NHCO | 4-indanyl | 0 | - |
| 476 | (CH2)30H | (II) | NHCO | 4-indanyl | 0 | - |
| 477 | (CH2)40H | (II) | NHCO | 4-indanyl | 0 | - |
| 478 | (CH2)20H | (II) | NHCO | 5,6,7,8-tetrahydro -1-naphthyl | 0 | - |
| 479 | (CH2)30H | (II) | NHCO | 5,6,7,8-tetrahydro -1-naphthyl | 0 | - |
| 480 | (CH2)40H | (II) | NHCO | 5,6,7,8-tetrahydro -1-naphthyl | 0 | - |

In the following table, $R^2$, X, Ar, m, n, $R^4$ and Y are as defined in the general formula (I). In this case, in the general formula (I), $R^1$ is a methyl group and $R^3$ is hydrogen.

Table 18

| Compound | $R^1$ | $R^2$ | X | Ar | m or n | Y |
|---|---|---|---|---|---|---|
| 481 | (III) | NHCO | 2,3-dimethyl phenyl | 1 | Me | $NH_2$ |

Table 18 (continued)

| Compound | $R^1$ | $R^2$ | X | Ar | m or n | Y |
|---|---|---|---|---|---|---|
| 482 | (III) | NHCO | 2,3-dimethyl phenyl | 1 | Et | $NH_2$ |
| 483 | (III) | NHCO | 2,3-dimethyl phenyl | 1 | Pr | $NH_2$ |
| 484 | (III) | NHCO | 2,3-dimethyl phenyl | 1 | Bu | $NH_2$ |
| 485 | (III) | NHCO | 2,3-dimethyl phenyl | 1 | i-Bu | $NH_2$ |
| 486 | (III) | NHCO | 2,3-dimethyl phenyl | 1 | H | $NH_2$ |
| 487 | (III) | NHCO | 1-naphthyl | 1 | Me | $NH_2$ |
| 488 | (III) | NHCO | 1- naphthyl | 1 | Et | $NH_2$ |
| 489 | (III) | NHCO | 1- naphthyl | 1 | Pr | $NH_2$ |
| 490 | (III) | NHCO | 1- naphthyl | 1 | Bu | $NH_2$ |
| 491 | (III) | NHCO | 1- naphthyl | 1 | i-Bu | $NH_2$ |
| 492 | (III) | NHCO | 4-indanyl | 1 | Me | $NH_2$ |
| 493 | (III) | NHCO | 4-indanyl | 1 | Et | $NH_2$ |
| 494 | (III) | NHCO | 4-indanyl | 1 | Pr | $NH_2$ |
| 495 | (III) | NHCO | 4-indanyl | 1 | Bu | $NH_2$ |
| 496 | (III) | NHCO | 4-indanyl | 1 | i-Bu | $NH_2$ |
| 497 | (III) | NHCO | 2,3-dimethyl phenyl | 1 | Me | OH |
| 498 | (III) | NHCO | 2,3-dimethyl phenyl | 1 | Et | OH |
| 499 | (III) | NHCO | 2,3-dimethyl phenyl | 1 | Pr | OH |
| 500 | (III) | NHCO | 2,3-dimethyl phenyl | 1 | Bu | OH |
| 501 | (III) | NHCO | 2,3-dimethyl phenyl | 1 | i-Bu | OH |
| 502 | (II) | NHCO | 1- naphthyl | 0 | Me | - |
| 503 | (III) | NHCO | 4-indanyl | 1 | Et | OH |
| 504 | (III) | NHCO | 4-indanyl | 1 | Pr | OH |
| 505 | (III) | NHCO | 4-indanyl | 1 | Bu | OH |
| 506 | (II) | NHCO | 2,3-dimethyl phenyl | 0 | Me | - |

The uracil derivative represented by the general formula (I) may be synthesized by combining a 1-substituted-2,4(1H,3H)-3-(substituted or unsubstituted)-pyrimidinedione derivative, an intermediate for the synthesis with a corresponding antioxidant unit under suitable reaction conditions. Specifically, a compound of the formula (I) in which X = NHCO may be synthesized by amidation reaction of a 5,6-diamino-3-(substituted or unsubstituted)-1-substituted-2,4(1H,3H)-pyrimidinedione derivative with a corresponding carboxylic acid. For example, an acid halide converted from the carboxylic acid may be used in the amidation reaction. It is also possible to react the carboxylic acid with an activated reagent such as a carbodiimide or a phosphoryl halide and use the reaction product in the amidation reaction. A compound of the formula (I) in which X = $NHCH_2$ may be synthesized by synthesizing a corresponding compound of the formula (I) in which X = NHCO by the process described above, and reducing this compound with diborane.

A compound of the formula (I) in which X = CONH and a compound of the formula (I) in which X = CO may be synthesized by a process based on the method of Bernier et al. (Bull. Soc. Chim. Fr., 1976, 616). That is, the compound in which X = CONH may be synthesized by directly reacting a 6-amino-3-(substituted or unsubstituted)-1-substituted-2,4(1H,3H)-pyrimidinedione derivative with an isocyanate. The compound in which X = CO may be synthesized by directly reacting a 6-amino-3-(substituted or unsubstituted)-1-substituted-2,4(1H,3H)-pyrimidinedione with an acid halide. A compound of the formula (I) in which X = $CH_2NH$ may be synthesized by synthesizing a corresponding compound of the formula (I) in which X = CONH by the method described above and reducing this compound with diborane.

3-(Substituted or unsubstituted)-1-substituted-5,6-diamino-2,4(1H,3H)-pyrimidinedione, an intermediate for

the synthesis having a 2,4(1H,3H)-pyrimidinedione ring, may be synthesized, for example, by the same method as described in JP-A-8-109171 and Japanese Patent No. 3093170.

A 1,3-substituted-2,4(1H,3H)-pyrimidinedione in which $R^1$ is a lower alkyl group may be obtained by converting the amino group at the 6-position to a hydroxyl group with hydrochloric acid, replacing the hydroxyl group with a chloro group, for example, by the method of Senda et al. (Chem. Pharm. Bull., 1974, 22, 189), and then reacting the resulting compound with any of various alkylamines. Thereafter, an amino group may be introduced into the 5-position by the same method as described above.

When $R^2$ represents the general formula (II) and X is NHCO, a carboxylic acid used as a starting material may be synthesized, for example, by the similar method of Lars et al. (Tetrahedron, 1970, 26, 879) (m = 0) or the process disclosed in JP-A-7-215959 (m = 1). When $R^2$ represents the general formula (II) and X is CONH, an isocyanate used as a starting material may be synthesized by reacting the carboxylic acid obtained by the above with diphenylphosphoryl azide.

When $R^2$ represents the general formula (III) and X is NHCO, a starting material may be synthesized by protecting the phenolic hydroxyl group or amino group of commercial 2,3,5-trimethyl-1,4-hydroquinone and 2,3,5-trimethyl-4-hydroxyaniline (Journal of American Chemical Society, 1939, 61, 765), respectively, by conventional methods, reacting them with a haloalkanoate, and then hydrolyzing the reaction product.

As pharmaceutically acceptable salts of derivatives having a functional group capable of forming the salt, among the uracil derivatives represented by the general formula (I), there are exemplified hydrochloride, sulfate, acetate, succinate, sodium salt, potassium salt, calcium salt and ammonium salt. These salts may be obtained by mixing the uracil derivative with an acid or a base, followed by purification by a conventional method such as recrystallization.

The present invention includes hydrates and solvates (e.g. ethanol solvates) of the uracil derivatives represented by the general formula (I) or pharmaceutically acceptable salts thereof. In addition, the present invention includes all tautomers of the uracil derivatives represented by the general formula (I), all stereoisomers (e.g. optical isomers) which the uracil derivatives have, and all crystal forms of the uracil derivatives.

The therapeutic agent for allergic diseases and pruritus of the present invention may be used in any of dosage forms such as oral preparations (e.g. tablets, capsules and powders), injections, external preparations and the like. For example, the uracil derivative or pharmaceutically acceptable salt thereof of the present invention may be formulated into an ointment by mixing with an ointment base such as vaseline. The uracil derivative or pharmaceutically acceptable salt thereof of the present invention may also be formulated into tablets by mixing with conventional additives such as excipients (e.g. lactose and starch), lubricants (e.g. magnesium stearate and talc) and the like.

The dose of the therapeutic agent for allergic diseases and pruritus of the present invention is properly determined depending on the sex, age and body weight of a patient, a disease to be treated, the symptom of the patient, and the like. For example, an ointment containing 0.01 to 10% of the active ingredient may be applied to an affected part once to several times per day in the case of a skin disease such as atopic dermatitis, contact dermatitis, psoriasis, pruritus in dialysis, or the like. When used in the form of an oral preparation such as tablets, capsules, powder or the like, the therapeutic agent of the present invention may be administered in a dose of 0.01 to 100 mg/kg per day in one portion or several portions.

## EXAMPLES

The present invention is concretely illustrated with the following examples, which should not be construed as limiting the scope of the invention.

### Reference Example 1: 6-amino-3-methyl-1-(1-naphthyl)uracil

A 40% aqueous methylamine solution (3.82 mL) was added to a solution of 1-naphthyl isocyanate (5.01 g, 29.6 mmol) in acetonitrile (100 mL) under ice-cooling. The temperature was raised to room temperature and the reaction was carried out for 5 hours. Then, the precipitates were filtered with a Kiriyama funnel under reduced pressure and washed with ether, and the thus obtained solid was dried at 40°C for 10 hours under reduced pressure to obtain 5.20 g of N-methyl-N'-(1-naphthyl)urea (yield: 88%). Cyanoacetic acid (6.37 g, 74.9 mmol) and then acetic anhydride (7.65 g, 74.9 mmol) were added to a suspension of the obtained urea (5.00 g, 25.0 mmol) in ethyl acetate (200 mL), and the resulting mixture was heated under reflux for 4 hours. After the temperature was lowered to room temperature, the reaction mixture was concentrated under reduced pressure and then water (150 mL) was added to the residue to precipitate a solid. The precipitated solid was filtered with a Kiriyama funnel under reduced pressure and the thus obtained solid is added to water (150 mL). A 1N aqueous sodium hydroxide solution was added thereto until the pH became 10, and the resulting mixture was vigorously stirred at room temperature for 1 hour. This mixture was filtered with a Kiriyama funnel under reduced pressure and washed with ether, and the thus obtained solid was dried at 40°C for 15 hours under reduced pressure to obtain 5.00 g of the title compound (yield: 75%).

Reference Example 2: 6-amino-3-methyl-1-(1-naphthyl)-5-nitrosouracil

The compound obtained in Reference Example 1 (1.00 g, 4.12 mmol) was added to an aqueous solution (2 mL) of sodium nitrite (426 mg, 6.18 mmol), followed by adding thereto concentrated hydrochloric acid (515 μL) under ice-cooling, and the resulting mixture was stirred for 10 hours. The pH of the reaction mixture was adjusted to 7 with a 5% aqueous sodium hydrogencarbonate solution. The precipitates were filtered with a Kiriyama funnel under reduced pressure and dried at 40°C for 15 hours under reduced pressure to obtain 1.03 g of the title compound (yield 84%).

Reference Example 3: 5,6-diamino-3-methyl-1-(1-naphthyl)uracil

A suspension of the compound obtained in Reference Example 2 (1.03 g, 3.48 mmol) and 5% palladium/carbon (100 mg) in methanol (10 mL) was stirred under a hydrogen atmosphere for 10 hours. After the catalyst was filtered off, the solvent was distilled off under reduced pressure and diethyl ether (10 mL) was added to the residue. The precipitates were filtered with a Kiriyama funnel under reduced pressure and dried at 40°C for 10 hours under reduced pressure to obtain 583 mg of the title compound (yield 59%).

Reference Example 4: 2,3,6-trimethyl-4-(pivaloyloxy)phenol

Pyridine (17.0 mL, 210 mmol) was added to a suspension of 2,3,6-trimethylhydroquinone (10.0 g, 65.7 mmol) in methylene chloride (70 mL), and then a solution of pivaloyl chloride (8.1 mL, 65.7 mmol) in methylene chloride (60 mL) was slowly dropped thereinto under ice-cooling. After completion of the dropping, the temperature was raised to room temperature and the mixture was stirred for 20 hours, followed by adding thereto acetic acid (3.76 mL) and water (150 mL). The organic layer was washed with water and then a 10% aqueous sodium chloride solution and dried over anhydrous sodium sulfate. The solvent was distilled off under reduced pressure and the residue was purified by a silica gel column chromatography to obtain 10.4 g of the title compound (yield: 67%).

Reference Example 5: 4-benzyloxy-2,3,5-trimethylphenol

After 60% sodium hydride (609 mg, 15.2 mmol) was washed several times with n-hexane, it was suspended in dimethylformamide (20 mL) and a solution of the compound obtained in Reference Example 4 (3.00 g, 12.7 mmol) in dimethylformamide (5 mL) was added dropwise thereto under ice-cooling. After completion of the dropwise addition, the resulting mixture was stirred for 30 minutes, followed by adding thereto a solution of benzyl bromide (2.82 g, 16.5 mmol) in dimethylformamide (5 mL), and the resulting mixture was stirred at room temperature for 4 hours. The solvent was distilled off under reduced pressure and a 5% aqueous citric acid solution was added to the residue, followed by extraction with diisopropyl ether. The organic layer was washed with water and then a 10% aqueous sodium chloride solution and dried over anhydrous sodium sulfate. The solvent was distilled off under reduced pressure and the residue was purified by a silica gel column chromatography to obtain 2.64 g of 1-benzyloxy-4-(pivaloyloxy)-2,3,6-trimethylbenzene (yield: 64%). Then, to a solution of this compound (2.60 g, 7.96 mmol) in dimethylformamide (10 mL) was added a suspension of potassium hydroxide (1.79 g, 31.8 mmol) in methanol under ice-cooling, and the temperature was raised to room temperature, followed by stirring for 12 hours.

The solvent was distilled off under reduced pressure and a 5% aqueous citric acid solution was added to the residue, followed by extraction with diisopropyl ether. The organic layer was washed successively with a 5% aqueous sodium hydrogencarbonate solution, water and a 10% aqueous sodium chloride solution and dried over anhydrous sodium sulfate. The solvent was distilled off under reduced pressure and the residue was dissolved in methanol, and then water was added thereto to precipitate a solid. The solid was filtered with a Kiriyama funnel under reduced pressure and washed with n-hexane, and the thus obtained solid was dried at 40°C for 12 hours under reduced pressure to obtain 1.68 g of the title compound as a white solid (yield: 87%).

Reference Example 6: (4-benzyloxy-2,3,5-trimethyl)phenoxyacetic acid

Under ice-cooling, 60% sodium hydride (73 mg, 1.82 mmol) washed several times with n-hexane was slowly added to a solution of the compound obtained in Reference Example 5 (400 mg, 1.65 mmol) in dimethylformamide (16 mL). The temperature was raised to room temperature and the mixture was stirred for 30 minutes, and then a solution of ethyl bromoacetate (202 μL, 1.82 mmol) in dimethylformamide (2 mL) was added thereto, followed by stirring at room temperature for 3 hours. The solvent was distilled off under reduced pressure and a 5% aqueous citric acid solution was added to the residue, followed by extraction with diisopropyl ether. The organic layer was washed with water and then a 10% aqueous sodium chloride solution and dried over anhydrous sodium sulfate. The solvent was distilled off under reduced pressure and the residue was purified by a silica gel column chromatography to obtain 495

mg of ethyl (4-benzyloxy-2,3,5-trimethyl)phenoxyacetate (yield: 91%). Then, to a solution of this ester (477 mg, 1.45 mmol) in ethanol (20 mL) was added a 1N aqueous sodium hydroxide solution (5 mL), and the resulting mixture was stirred at room temperature for 8 hours. After the pH was adjusted to 7 with 1N hydrochloric acid under ice-cooling, the solvent was distilled off under reduced pressure. Water was added to the residue to precipitate a solid and the solid was filtered with a Kiriyama funnel under reduced pressure. The solid thus obtained was suspended in ethanol/water at room temperature for 1 hour and the suspension was filtered with a Kiriyama funnel under reduced pressure. The precipitate was dried at 40°C for 15 hours under reduced pressure to obtain 415 mg of the title compound (yield: 95%).

Reference Example 7: tert-butyl 4-hydroxy-2,3,6-trimethylphenylcarbamate

Di-tert-butyl dicarbonate (1.51 g, 6.91 mmol) was added to a solution of 4-amino-2,3,5-trimethylphenol (950 mg, 6.28 mmol) in tetrahydrofuran (12 mL) and the resulting mixture was heated under reflux for 2 hours. After the temperature was lowered to room temperature, water was added to the mixture, followed by extraction with diisopropyl ether. The organic layer was washed with water and then a 10% aqueous sodium chloride solution and dried over anhydrous sodium sulfate. The solvent was distilled off under reduced pressure and the residue was purified by a silica gel column chromatography to obtain 1.40 g of the title compound (yield: 89%).

Reference Example 8: tert-butyl 4-(carboxymethoxy)-2,3,6-trimethylphenylcarbamate

The title compound was obtained by the same process as in Reference Example 6 except for using the compound obtained in Reference Example 7, as a starting material in place of the compound obtained in Reference Example 5.

Reference Example 9: 6-amino-5-(6-benzyloxy-2,2,5,7-tetramethyl-3-oxaindan-4-carboxamide)-3-methyl-1-(1-naphthyl)uracil

Triethylamine (77 μL, 0.55 mmol) and diphenylphosphoryl chloride (148 mg, 0.55 mmol) were added to a solution of 6-benzyloxy-2,2,5,7-tetramethyl-3-oxaindan-4-carboxylic acid (180 mg, 0.55 mmol) in ethyl acetate (6 mL) under ice-cooling, and the resulting mixture was stirred for 1 hour. Subsequently, the compound obtained in Reference Example 3 (142 mg, 0.50 mmol) and then triethylamine (77 μL, 0.55 mmol) were added thereto, followed by stirring at room temperature for 5 hours. The solvent was distilled off under reduced pressure and water was added to the residue, followed by extraction with methylene chloride. The organic layer was washed successively with a 5% aqueous sodium hydrogencarbonate solution, water and a 10% aqueous sodium chloride solution and dried over anhydrous sodium sulfate. The solvent was distilled off under reduced pressure and the residue was purified by a silica gel column chromatography to obtain 224 mg of the title compound (yield: 69%).

Reference Example 10: 6-amino-5-(6-benzyloxy-2,2,5,7-tetramethylchroman-8-carboxamide)-3-methyl-1-(1-naphthyl)uracil

The title compound was obtained by the same process as in Reference Example 9 except for using 6-benzyloxy-2,2,5,7-tetramethylchroman-8-carboxylic acid as a starting material in place of 6-benzyloxy-2,2,5,7-tetramethyl-3-oxaindan-4-carboxylic acid.

Reference Example 11: 6-amino-5-[(4-benzyloxy-2,3,5-trimethyl)phenoxy]acetamide-3-methyl-1-(1-naphthyl)uracil

To a solution of the compound obtained in Reference Example 3 (230 mg, 0.81 mmol) and the compound obtained in Reference Example 6 (243 mg, 0.81 mmol) in dimethylformamide (6 mL) were added 1-hydroxybenzotriazole (120 mg, 0.89 mmol) and then N-ethyl-N'-[3-(dimethylamino)propyl]carbodiimide (171 mg, 0.89 mmol), and the resulting mixture was stirred at room temperature for 10 hours. The solvent was distilled off under reduced pressure and a 5% aqueous sodium hydrogencarbonate solution was added to the residue, followed by extraction with methylene chloride. The organic layer was washed successively with a 5% aqueous citric acid solution, water and a 10% aqueous sodium chloride solution and dried over anhydrous sodium sulfate. The solvent was distilled off under reduced pressure and the residue was purified by a silica gel column chromatography to obtain 242 mg of the title compound (yield: 53%).

Reference Example 12: 6-amino-5-[[4-(tert-butoxycarbonyl-amino)-2,3,5-trimethyl]phenoxy]acetamide-3-methyl-1-(1-naphthyl)uracil

The title compound was obtained by the same process as in Reference Example 15 except for using the

compound obtained in Reference Example 8, as a starting material in place of the compound obtained in Reference Example 6.

Reference Example 13: 6-amino-5-(6-benzyloxy-2,2,5,7- tetramethyl-3-oxaindan-4-yl)aminocarbonyl-3-methyl-1-(1-naphthyl)uracil

Triethylamine (542 µL, 3.89 mmol) and diphenylphosphoryl azide (1.07 g, 3.89 mmol) were added to a solution of 6-benzyloxy-2,2,5,7-tetramethyl-3-oxaindan-4-carboxylic acid (1.00 g, 3.54 mmol) in toluene (20 mL) under ice-cooling, and the resulting mixture was stirred for 2 hours. Thereafter, the reaction temperature was raised to 90°C and the mixture was stirred for another 3 hours and then cooled to room temperature to prepare a solution of an isocyanate corresponding to the starting carboxylic acid in toluene. On the other hand, 60% sodium hydride (113 mg, 2.83 mmol) washed several times with n-hexane was slowly added to a solution of the compound obtained in Reference Example 1 (757 mg, 2.83 mmol) in dimethylformamide (20 mL) under ice-cooling. Subsequently, the above-mentioned solution of the isocyanate in toluene was added thereto and the reaction was carried out at room temperature for 10 hours. The solvent was distilled off under reduced pressure and a 1N aqueous sodium hydroxide solution was added to the residue, followed by extraction with methylene chloride. The organic layer was washed with water and then a 10% aqueous sodium chloride solution and dried over anhydrous sodium sulfate. The solvent was distilled off under reduced pressure and the residue was purified by a silica gel column chromatography to obtain 250 mg of the title compound (yield: 15%).

Reference Example 14: 6-amino-5-(6-benzyloxy-2,2,5,7-tetramethyl-3-oxaindan-4-yl)carbonyl-3-methyl-1-(1-naphthyl)uracil

A solution of oxalyl chloride (170 µL, 1.95 mmol) in methylene (5 mL) chloride was added to a solution of 6-benzyloxy-2,2,5,7-tetramethyl-3-oxaindan-4-carboxylic acid (1.00 g, 3.54 mmol) in methylene chloride (15 mL), and the resulting mixture was stirred under ice-cooling for 2 hours. The temperature was raised to room temperature to prepare a solution of an acid chloride corresponding to the starting carboxylic acid in methylene chloride. On the other hand, 60% sodium hydride (170 mg, 4.25 mmol) washed several times with n-hexane was slowly added to a solution of the compound obtained in Reference Example 1 (1.14 g, 4.25 mmol) in dimethylformamide (15 mL) under ice-cooling. Subsequently, the above-mentioned solution of the acid chloride in methylene chloride was added thereto and the reaction was carried out at room temperature for 10 hours. After the solvent was distilled off under reduced pressure, water was added to the residue and the precipitates were filtered under reduced pressure. The thus obtained solid was purified by a silica gel column chromatography to obtain 650 mg of the title compound (yield: 32%).

Example 1: 6-amino-5-(6-hydroxy-2,2,5,7-tetramethyl-3-oxaindan-4-carboxamide)-3-methyl-1-(1-naphthyl)uracil (compound 1)

Concentrated hydrochloric acid (28 µL) was added to a suspension of the compound obtained in Reference Example 9 (200 mg, 0.34 mmol) and 5% palladium/carbon (20 mg) in methanol, and the resulting mixture was stirred under a hydrogen atmosphere for 15 hours. After the catalyst was filtered off, the solvent was distilled off under reduced pressure and the residue was purified by a silica gel column chromatography to obtain 140 mg of the title compound (yield: 82%).
1H NMR (DMSO-d6)δ 8.76 (s, 1H), 8.18-8.10 (m, 2H), 7.75-7.55 (m, 6H), 5.91 (br s, 2H), 3.20 (s, 3H), 2.86 (s, 2H), 2.15 (s, 3H), 2.05 (s, 3H), 1.29 (s, 3H), 1.26 (s, 3H).

Example 2: 6-amino-5-(6-hydroxy-2,2,5,7-tetramethyl-chroman-8-carboxamide)-3-methyl-1-(1-naphthyl)uracil (compound 2)

The title compound was obtained by the same process as in Example 1.
1H NMR (CDCl3)δ 8.08-7.97 (m, 2H), 7.77-7.75 (m, 1H), 7.65-7.60 (m, 4H), 7.24 (s, 1H), 4.99 (br s, 2H), 3.69 (s, 1H), 3.41 (s, 3H), 2.62 (t, J = 6.8 Hz, 2H), 2.29 (s, 3H), 2.14 (s, 3H), 1.78 (t, J = 6.8 Hz, 2H), 1.22 (s, 3H), 1.21 (s, 3H).

Example 3: 6-amino-5-(6-hydroxy-2,2,5,7-tetramethyl-chroman-8-carboxamide)-1-(1-naphthyl)uracil (compound 10)

The title compound was obtained by the same process as in Example 1.
1H NMR (DMSO-d6)δ 10.93 (s, 1H), 8.74 (s, 1H), 8.16-8.09 (m, 2H), 7.71-7.62 (m, 6H), 5.69 (br s, 2H), 2.51-2.50 (m, 2H), 2.14 (s, 3H), 2.06 (s, 3H), 1.70-1.67 (m, 2H), 1.09 (s, 3H), 1.08 (s, 3H).

Example 4: 6-amino-5-[(4-hydroxy-2,3,5-trimethyl)phenoxy]acetamide-3-methyl-1-(1-naphthyl)uracil (compound 3)

The title compound was obtained by the same process as in Example 1.
1H NMR (DMSO-d6)δ 8.44 (s, 1H), 8.14-8.08 (m, 2H), 7.70-7.54 (m, 6H), 6.53 (s, 1H), 6.13 (br s, 2H), 4.49 (s, 2H), 3.18 (s, 3H), 2.13 (s, 3H), 2.12 (s, 3H), 2.09 (s, 3H).

Example 5: 6-amino-5-[(4-amino-2,3,5-trimethyl)phenoxy]acetamide-3-methyl-1-(1-naphthyl)uracil (compound 4)

To a suspension of the compound obtained in Reference Example 12 (150 mg, 0.26 mmol) in ethyl acetate (5 mL) were added 4N hydrochloric acid/1,4-dioxane (1.2 mL) and acetic acid (3 mL), and the resulting mixture was stirred at room temperature for 2 hours. The solvent was distilled off under reduced pressure and a 5% aqueous sodium hydrogencarbonate solution was added to the residue, followed by extraction with methylene chloride. The organic layer was washed with water and then a 10% aqueous sodium chloride solution and dried over anhydrous sodium sulfate. The solvent was distilled off under reduced pressure and the residue was purified by a silica gel column chromatography to obtain 94 mg of the title compound (yield: 76%).
1H NMR (DMSO-d6)δ 8.41 (s, 1H), 8.14-8.08 (m, 2H), 7.70-7.56 (m, 5H), 6.61 (s, 1H), 6.12 (br s, 2H), 4.43 (s, 2H), 4.12 (br s, 2H), 3.18 (s, 3H), 2.12 (s, 3H), 2.06 (s, 3H), 2.01 (s, 3H).

Example 6: 6-amino-5-[[(6-hydroxy-2,2,5,7-tetramethyl-3-oxaindan-4-yl)methyl]amino]-3-methyl-1-(1-naphthyl)uracil (compound 15)

A borane-methyl sulfide complex (900 μL) was added to a solution of the compound obtained in Example 1 (950 mg, 1.90 mmol) in tetrahydrofuran (38 mL) under ice-cooling, and the resulting mixture was heated under reflux for 12 hours. Under ice-cooling, the reaction mixture was adjusted to pH 8 with a 1N aqueous sodium hydroxide solution and then extracted with methylene chloride. The organic layer was washed with water and then a 10% aqueous sodium chloride solution and dried over anhydrous sodium sulfate. The solvent was distilled off under reduced pressure and the residue was purified by a silica gel column chromatography to obtain 285 mg of the title compound (yield: 31%).
1H NMR (DMSO-d6)δ 8.12-8.06 (m, 2H), 7.68-7.42 (m, 7H), 5.74 (br s, 2H), 3.85-3.79 (m, 1H), 3.72-3.66 (m, 1H), 2.95 (s, 3H), 2.83 (s, 2H), 2.19 (s, 3H), 2.01 (s, 3H), 1.30 (s, 3H), 1.27 (s, 3H).

Example 7: 6-amino-5-(6-hydroxy-2,2,5,7-tetramethyl-3-oxaindan-4-yl)aminocarbonyl-3-methyl-1-(1-naphthyl)uracil (compound 16)

The title compound was obtained by the same process as in Example 1 except for using the compound of Reference Example 13.
1H NMR (DMSO-d6)δ 8.50 (br s, 1H), 8.11-8.09 (m, 2H), 7.62-7.45 (m, 7H), 6.63 (s, 1H), 3.17 (s, 3H), 2.82 (s, 2H), 2.00 (s, 3H), 1.77 (s, 3H), 1.28 (s, 6H).

Example 8: 6-amino-5-(6-hydroxy-2,2,5,7-tetramethyl-3-oxaindan-4-yl)carbonyl-3-methyl-1-(1-naphthyl)uracil (compound 17)

The title compound was obtained by the same process as in Example 1 except for using the compound of Reference Example 14.
1H NMR (DMSO-d6)δ 8.67 (s, 1H), 8.11-8.06 (m, 2H), 7.83 (s, 1H), 7.73-7.62 (m, 5H), 6.65 (s, 1H), 3.20 (s, 3H), 2.65 (s, 2H), 1.95 (s, 6H), 0.87 (s, 3H), 0.75 (s, 3H).

Example 9: 6-amino-3-ethyl-5-(6-hydroxy-2,2,5,7-tetramethylchroman-8-carboxamide)-1-(6-methoxypyridin-3-yl) uracil (compound 439)

The title compound was obtained by the same process as in Example 1.
1H NMR (DMSO-$d_6$)δ 8.79 (s, 1H), 8.20 (d, J = 2.4 Hz, 1H), 7.73-7.78 (m, 2H), 6.99 (d, J = 8.9 Hz, 1H), 5.85 (br s, 2H), 3.94 (s, 3H), 3.83 (q, J = 6.9 Hz, 2H), 2.54 (m, 2H), 2.14 (s, 3H), 2.07 (s, 3H), 1.71 (t, J = 6.9 Hz, 3H), 1.07-1.15 (m, 8H).

Example 10: 6-amino-3-ethyl-5-(6-hydroxy-2,2,5,7-tetramethylchroman-8-carboxamide)-1-(2-methoxypyridin-3-yl) uracil (compound 433)

The title compound was obtained by the same process as in Example 1.

1H NMR (DMSO-$d_6$)δ 8.76 (s, 1H), 8.35 (m, 1H), 7.82 (q, J = 1.6 Hz, 1H), 7.73 (s, 1H), 7.16-7.21 (m, 1H), 5.84 (br s, 2H), 3.90 (s, 3H), 3.82 (q, J = 6.9 Hz, 2H), 2.54 (m, 2H), 2.14 (s, 3H), 2.06 (s, 3H), 1.72 (t, J = 6.9 Hz, 3H), 1.07-1.15 (m, 8H).

Example 11: 6-amino-1-(6-chloropyridin-3-yl)-3-ethyl-5-(6-hydroxy-2,2,5,7-tetramethylchroman-8-carboxamide)uracil (compound 457)

The title compound was obtained by the same process as in Example 1.
1H NMR (DMSO-$d_6$)δ 8.84 (s, 1H), 8.52 (d, J = 2.4 Hz, 1H), 7.99-8.03 (m, 1H), 7.77 (s, 1H), 7.74 (s, 1H), 5.88 (br s, 2H), 3.82 (q, J = 6.9 Hz, 2H), 2.54 (m, 2H), 2.14 (s, 3H), 2.07 (s, 3H), 1.72 (t, J = 6.9 Hz, 3H), 1.07-1.14 (m, 8H).

Example 12: 6-amino-3-ethyl-5-(6-hydroxy-2,2,5,7-tetramethyl-3-oxaindan-4-carboxamide)-1-(1-naphthyl)uracil (compound 19)

The title compound was obtained by the same process as in Example 1.
1H NMR (DMSO-d6)δ 1.13 (3H, t, J = 7.0 Hz), 1.25 (3H, s), 1.29 (3H, s), 2.05 (3H, s), 2.15 (3H, s), 2.85 (2H, s), 3.86 (2H, q, J = 7.0 Hz), 5.91 (2H, brs), 7.53-7.55 (1H, m), 7.62-7.72 (4H, m), 7.76 (1H, s), 8.10-8.17 (2H, m), 8.74 (1H, s); MS m/z 515$(M+H)^+$

Example 13: 6-amino-3-propyl-5-(6-hydroxy-2,2,5,7-tetramethyl-3-oxaindan-4-carboxamide)-1-(1-naphthyl)uracil (compound 20)

The title compound was obtained by the same process as in Example 1.
1H NMR (DMSO-d6)δ 0.86 (3H, t, J = 7.4 Hz), 1.25 (3H, s), 1.29 (3H, s), 1.54-1.60 (2H, m), 2.05 (3H, s), 2.15 (3H, s), 2.85 (2H, s), 3.78 (2H, t, J = 7.4 Hz), 5.92 (2H, brs), 7.52-7.54 (1H, m), 7.62-7.72 (4H, m), 7.75 (1H, s), 8.10-8.17 (2H, m), 8.74 (1H, s); MS m/z 529$(M+H)^+$

Example 14: 6-amino-3-butyl-5-(6-hydroxy-2,2,5,7-tetramethyl-3-oxaindan-4-carboxamide)-1-(1-naphthyl)uracil (compound 21)

The title compound was obtained by the same process as in Example 1.
1H NMR (DMSO-d6)δ 0.89 (3H, t, J = 7.3 Hz), 1.25-1.34 (8H, m), 1.50-1.57 (2H, m), 2.05 (3H, s), 2.15 (3H, s), 2.85 (2H, s), 3.81 (2H, t, J = 7.3Hz), 5.92 (2H, brs), 7.51-7.54 (1H, m), 7.62-7.72 (4H, m), 7.75 (1H, s), 8.10-8.17 (2H, m), 8.74 (1H, s); MS m/z 543$(M+H)^+$

Example 15: 6-methylamino-3-methyl-5-(6-hydroxy-2,2,5,7-tetramethyl-3-oxaindan-4-carboxamide)-1-(1-naphthyl) uracil (compound 502)

The title compound was obtained by the same process as in Example 1.
1H NMR (DMSO-d6)δ 1.27 (6H, s), 2.06 (3H, s), 2.31 (3H, s), 2.82 (3H, d, J = 5.1Hz), 2.84 (2H, s), 3.18 (3H, s), 5.29-5.33 (1H, m), 7.55-7.68 (6H, m), 8.08-8.14 (2H, m), 8.75 (1H, s); MS m/z 515$(M+H)^+$

Example 16: 6-amino-3-methyl-5-(6-hydroxy-2,2,5,7-tetramethyl-3-oxaindan-4-carboxamide)-1-(2,3-dimethylphenyl) uracil (compound 301)

The title compound was obtained by the same process as in Example 1.
1H NMR (DMSO-d6)δ 1.34 (6H, s), 1.99 (3H, s), 2.06 (3H, s), 2.13 (3H, s), 2.34 (3H, s), 2.88 (2H, s), 3.17 (3H, s), 5.83 (2H, brs), 7.12 (1H, d, J = 7.5 Hz), 7.26-7.30 (1H, m), 7.35 (1H, d, J = 7.4 Hz), 7.75 (1H, brs), 8.71 (1H, s); MS m/z 479$(M+H)^+$

Example 17: 6-amino-3-ethyl-5-(6-hydroxy-2,2,5,7-tetramethyl-3-oxaindan-4-carboxamide)-1-(2,3-dimethylphenyl) uracil (compound 331)

The title compound was obtained by the same process as in Example 1.
1H NMR (DMSO-d6)δ 1.10 (3H, t, J = 7.0 Hz), 1.34 (6H, s), 1.98 (3H, s), 2.06 (3H, s), 2.13 (3H, s), 2.34 (3H, s), 2.88 (2H, s), 3.85 (2H, q, J = 7.0 Hz), 5.83 (2H, brs), 7.13 (1H, d, J = 7.5 Hz), 7.26-7.30 (1H, m), 7.35 (1H, d, J = 7.4 Hz), 7.74 (1H, s), 8.68 (1H, s); MS m/z 493$(M+H)^+$

Example 18: 6-amino-3-propyl-5-(6-hydroxy-2,2,5,7-tetramethyl-3-oxaindan-4-carboxamide)-1-(2,3-dimethylphenyl) uracil (compound 361)

The title compound was obtained by the same process as in Example 1.
1H NMR (DMSO-d6)δ 0.85 (3H, t, J = 7.4 Hz), 1.33 (3H, s), 1.34 (3H, s), 1.50-1.60 (2H, m), 1.98 (3H, s), 2.05 (3H, s), 2.13 (3H, s), 2.34 (3H, s), 2.87 (2H, s), 3.71-3.79 (2H, m), 5.83 (2H, brs), 7.13 (1H, d, J = 7.5 Hz), 7.26-7.30 (1H, m), 7.35 (1H, d, J = 7.4 Hz), 7.74 (1H, s), 8.68 (1H, s); MS m/z 507$(M+H)^+$

Example 19: 6-methylamino-3-methyl-5-(6-hydroxy-2,2,5,7-tetramethyl-3-oxaindan-4-carboxamide)-1-(2,3-dimethylphenyl)uracil (compound 506)

The title compound was obtained by the same process as in Example 1.
1H NMR (DMSO-d6)δ 1.30 (6H, s), 1.96 (3H, s), 2.05 (3H, s), 2.28 (3H, s), 2.32 (3H, s), 2.85 (2H, s), 2.89 (3H, d, J = 5.1Hz) 3.15 (3H, s), 5.07-5.10 (1H, m), 7.05 (1H, d, J = 7.5 Hz), 7.23-7.27 (1H, m), 7.32 (1H, d, J = 7.4 Hz), 7.65 (1H, s), 8.69 (1H, s); MS m/z 493$(M+H)^+$

Example 20: 6-amino-3-ethyl-5-(6-hydroxy-2,2,5,7-tetramethyl-3-oxaindan-4-carboxamide)-1-(4-indanyl)uracil (compound 91)

The title compound was obtained by the same process as in Example 1.
1H NMR (DMSO-d6)δ 1.10 (3H, t, J = 7.0 Hz), 1.34 (6H, s), 2.01-2.09 (5H, m), 2.13 (3H, s), 2.61-2.74 (2H, m), 2.88 (2H, s), 2.93-3.04 (2H, m), 3.83 (2H, q, J = 7.0 Hz), 5.88 (2H, brs), 7.12 (1H, d, J = 7.4 Hz), 7.31-7.35 (1H, m), 7.41 (1H, d, J = 7.3 Hz), 7.75 (1H, s), 8.67 (1H, s); MS m/z 505$(M+H)^+$

Example 21: 6-amino-3-propyl-5-(6-hydroxy-2,2,5,7-tetramethyl-3-oxaindan-4-carboxamide)-1-(4-indanyl)uracil (compound 121)

The title compound was obtained by the same process as in Example 1.
1H NMR (DMSO-d6)δ 0.85 (3H, t, J = 7.4 Hz), 1.34 (6H, s), 1.50-1.59 (2H, m), 2.01-2.09 (5H, m), 2.13 (3H, s), 2.60-2.74 (2H, m), 2.88 (2H, s), 2.93-3.05 (2H, m), 3.69-3.80 (2H, m), 5.88 (2H, brs), 7.11 (1H, d, J = 7.4 Hz), 7.31-7.35 (1H, m), 7.41 (1H, d, J = 7.4 Hz), 7.74 (1H, s), 8.67 (1H, s); MS m/z 519$(M+H)^+$

Example 22: 6-amino-3-methyl-5-(6-hydroxy-2,2,5,7-tetramethyl-3-oxaindan-4-carboxamide)-1-(5,6,7,8-tetrahydro-1-naphthyl)uracil (compound 181)

The title compound was obtained by the same process as in Example 1.
1H NMR (DMSO-d6)δ 1.34 (3H, s), 1.35 (3H, s), 1.70-1.77 (4H, m), 2.06 (3H, s), 2.13 (3H, s), 2.40-2.41 (2H, m), 2.81-2.82 (2H, m), 2.88 (2H, s), 3.16 (3H, s), 5.82 (2H, brs), 7.09-7.11 (1H, m), 7.25-7.31 (2H, m), 7.74 (1H, s), 8.70 (1H, s); MS m/z 505$(M+H)^+$

Example 23: 6-amino-3-ethyl-5-(6-hydroxy-2,2,5,7-tetramethyl-3-oxaindan-4-carboxamide)-1-(5,6,7,8-tetrahydro-1-naphthyl)uracil (compound 211)

The title compound was obtained by the same process as in Example 1.
1H NMR (DMSO-d6)δ 0.86 (3H, t, J = 6.8 Hz), 1.34 (6H, s), 1.71-1.74 (4H, m), 2.06 (3H, s), 2.13 (3H, s), 2.33-2.45 (2H, m), 2.76-2.82 (2H, m), 2.88 (2H, s), 5.82 (2H, brs), 7.09-7.12 (1H, m), 7.25-7.31 (2H, m), 7.74 (1H, s), 8.67 (1H, s); MS m/z 519$(M+H)^+$

Example 24: 6-amino-3-propyl-5-(6-hydroxy-2,2,5,7-tetramethyl-3-oxaindan-4-carboxamide)-1-(5,6,7,8-tetrahydro-1-naphthyl)uracil (compound 241)

The title compound was obtained by the same process as in Example 1.
1H NMR (DMSO-d6)δ 0.85 (3H, t, J = 7.4 Hz), 1.34 (6H, s), 1.50-1.59 (2H, m), 1.70-1.77 (4H, m), 2.05 (3H, s), 2.12 (3H, s), 2.28-2.45 (2H, m), 2.76-2.82 (2H, m), 2.88 (2H, s), 5.83 (2H, brs), 7.09-7.13 (1H, m), 7.21-7.31 (2H, m), 7.74 (1H, s), 8.67 (1H, s); MS m/z 533$(M+H)^+$

Example 25: 6-amino-5-[(4-amino-2,3,5-trimethyl)phenoxy]acetamide-3-ethyl-1-(1-naphthyl)uracil (compound 12)

The title compound was obtained by the same process as in Example 5.
1H NMR (DMSO-d6)δ 1.11 (3H, t, J = 6.9 Hz), 2.01 (3H, s), 2.06 (3H, s), 2.13 (3H, s), 3.81-3.88 (2H, m), 4.12 (2H, brs), 4.42 (2H, s), 6.12 (2H, brs), 6.61 (1H, s), 7.52-7.69 (5H, m), 8.08-8.13 (2H, m), 8.42 (1H, s); MS m/z 488$(M+H)^+$

Example 26: 6-amino-5-[(4-amino-2,3,5-trimethyl)phenoxy]acetamide-3-propyl-1-(1- naphthyl)uracil (compound 13)

The title compound was obtained by the same process as in Example 5.
1H NMR (DMSO-d6)δ 0.85 (3H, t, J = 7.5 Hz), 1.51-1.60 (2H, m), 2.01 (3H, s), 2.06 (3H, s), 2.13 (3H, s), 3.76 (2H, t, J = 7.3 Hz), 4.12 (2H, brs), 4.42 (2H, s), 6.12 (2H, brs), 6.60 (1H, s), 7.51-7.69 (5H, m), 8.08-8.13 (2H, m), 8.42 (1H, s); MS m/z 502$(M+H)^+$

Example 27: 6-amino-5-[(4-amino-2,3,5-trimethyl)phenoxy]acetamide-3-butyl-1-(1-naphthyl)uracil (compound 14)

The title compound was obtained by the same process as in Example 5.
1H NMR (DMSO-d6)δ 0.88 (3H, t, J = 7.3 Hz), 1.23-1.32 (2H, m), 1.48-1.55 (2H, m), 2.01 (3H, s), 2.06 (3H, s), 2.13 (3H, s), 3.79 (2H, t, J = 7.3 Hz), 4.20 (2H, brs), 4.42 (2H, s), 6.12-(2H, brs), 6.60 (1H, s), 7.52-7.69 (5H, m), 8.08-8.13 (2H, m), 8.42 (1H, s); MS m/z 516 $(M+H)^+$

Example 28: 6-methylamino-5-[(4-amino-2,3,5-trimethyl)phenoxy]acetamide-3-methyl-1-(1-naphthyl)uracil (compound 487)

The title compound was obtained by the same process as in Example 5.
1H NMR (DMSO-d6)δ 2.00 (3H, s), 2.04 (3H, s), 2.10 (3H, s), 2.60 (3H, d, J = 5.0 Hz), 4.13 (2H, brs), 4.42 (2H, s), 5.40-5.44 (2H, m), 6.60 (1H, s), 7.54-7.68 (5H, m), 8.07-8.13 (2H, m), 8.71 (1H, s); MS m/z 488 $(M+H)^+$

Example 29: 6-amino-5-[(4-amino-2,3,5-trimethyl)phenoxy]acetamide-3-methyl-1-(2,3-dimethylphenyl)uracil (compound 304)

The title compound was obtained by the same process as in Example 5.
1H NMR (DMSO-d6)δ 1.97 (3H, s), 2.01 (3H, s), 2.06 (3H, s), 2.12 (3H, s), 2.32 (3H, s), 3.15 (2H, s), 4.12 (2H, brs), 4.42 (2H, s), 6.00 (2H, brs), 6.60 (1H, s), 7.08 (1H, d, J = 7.6 Hz), 7.24-7.27 (1H, m), 7.32 (1H, d, J = 7.5 Hz), 8.38 (1H, s); MS m/z 452 $(M+H)^+$

Example 30: 6-methylamino-5-[(4-amino-2,3,5-trimethyl)phenoxy]acetamide-3-methyl-1-(2,3-dimethylphenyl)uracil (compound 481)

The title compound was obtained by the same process as in Example 5.
1H NMR (DMSO-d6)δ 1.95 (3H, s), 2.00 (3H, s), 2.04 (3H, s), 2.10 (3H, s), 2.31 (3H, s), 2.67 (3H, t, J = 5.0 Hz), 3.14 (3H, s), 4.13 (2H, brs), 4.40 (2H, s), 5.20-5.24 (1H, m), 6.59 (1H, s), 7.05 (1H, d, J = 7.6 Hz), 7.22-7.26 (1H, m), 7.32 (1H, d, J = 7.5 Hz), 8.66 (1H, s); MS m/z 466 $(M+H)^+$

Example 31: 6-ethylamino-5-[(4-amino-2,3,5-trimethyl)phenoxy]acetamide-3-methyl-1-(2,3-dimethylphenyl)uracil (compound 482)

The title compound was obtained by the same process as in Example 5.
1H NMR (DMSO-d6)δ 0.84 (3H, t, J = 7.1 Hz), 1.96 (3H, s), 2.00 (3H, s), 2.05 (3H, s), 2.10 (3H, s), 2.31 (3H, s), 3.04-3.12 (2H, m), 3.15 (3H, s), 4.13 (2H, brs), 4.92-4.95 (1H, m), 6.59 (1H, s), 7.07 (1H, d, J = 7.4 Hz), 7.23-7.27 (1H, m), 7.32 (1H, d, J = 7.4 Hz), 8.68 (1H, s); MS m/z 480 $(M+H)^+$

Example 32: 6-propylamino-5-[(4-amino-2,3,5-trimethyl)phenoxy]acetamide-3-methyl-1-(2,3-dimethylphenyl)uracil (compound 483)

The title compound was obtained by the same process as in Example 5.
1H NMR (DMSO-d6)δ 0.59 (3H, t, J = 7.4 Hz), 1.22-1.29 (2H, m), 1.96 (3H, s), 2.00 (3H, s), 2.05 (3H, s), 2.10 (3H, s), 2.31 (3H, s), 2.95-3.01 (2H, m), 3.15 (3H, s), 4.13 (2H, brs), 4.40 (2H, s), 4.86-4.89 (1H, m), 6.60 (1H, s), 7.08 (1H, d, J = 7.6 Hz), 7.24-7.28 (1H, m), 7.33 (1H, d, J = 7.4 Hz), 8.67 (1H, s); MS m/z 494 $(M+H)^+$

Example 33: 6-butylamino-5-[(4-amino-2,3,5-trimethyl)phenoxy]acetamide-3-methyl-1-(2,3-dimethylphenyl)uracil (compound 484)

The title compound was obtained by the same process as in Example 5.
1H NMR (DMSO-d6)δ 0.72 (3H, t, J = 7.3 Hz), 0.97-1.02 (2H, m), 1.19-1.24 (2H, m), 1.96 (3H, s), 2.00 (3H, s), 2.05 (3H, s), 2.10 (3H, s), 2.31 (3H, s), 2.98-3.04 (2H, m), 3.15 (3H, s), 4.14 (2H, brs), 4.40 (2H, s), 4.82-4.85 (1H, m), 6.60 (1H, s), 7.07 (1H, d, J = 7.5 Hz), 7.24-7.28 (1H, m), 7.33 (1H, d, J = 7.4 Hz), 8.66 (1H, s); MS m/z 508 $(M+H)^+$

Example 34: 6-amino-3-(2-hydroxyethyl)-5-(6-hydroxy-2,2,5,7-tetramethyl-3-oxaindan-4-carboxamide)-1-(2,3-dimethylphenyl)uracil (compound 466)

The title compound was obtained by the same process as in Example 1.
1H NMR (DMSO-d6)δ 1.34 (6H, s), 1.98 (3H, s), 2.05 (3H, s), 2.13 (3H, s), 2.33 (3H, s), 2.87 (2H, s), 3.43-3.51 (2H, m), 3.88 (2H, t, J = 6.8 Hz), 4.75 (1H, t, J = 5.6 Hz), 5.83 (2H, brs), 7.11 (1H, d, J = 7.2 Hz), 7.28 (1H, t, J = 8.0 Hz), 7.35 (1H, d, J = 7.2 Hz), 7.74 (1H, s), 8.69 (1H, s); MS m/z 509 (M+H)+

Example 35: 6-amino-3-(3-hydroxypropyl)-5-(6-hydroxy-2,2,5,7-tetramethyl-3-oxaindan-4-carboxamide)-1-(2,3-dimethylphenyl)uracil (compound 467)

The title compound was obtained by the same process as in Example 1.
1H NMR (DMSO-d6)δ 1.34 (6H, s), 1.65-1.72 (2H, m), 1.98 (3H, s), 2.05 (3H, s), 2.13 (3H, s), 2.34 (3H, s), 2.88 (2H, s), 3.38-3.43 (2H, m), 3.80-3.88 (2H, m), 4.43 (1H, t, J = 5.4 Hz), 5.91 (2H, brs), 7.12 (1H, d, J = 6.0 Hz), 7.28 (1H, t, J = 7.6 Hz), 7.42 (1H, d, J = 6.0 Hz), 7.74 (1H, s), 8.73 (1H, s); MS m/z 523 (M+H)+

Example 36: 6-amino-3-(2-methoxyethyl)-5-(6-hydroxy-2,2,5,7-tetramethyl-3-oxaindan-4-carboxamide)-1-(2,3-dimethylphenyl)uracil (compound 464)

The title compound was obtained by the same process as in Example 1.
1H NMR (DMSO-d6)δ 1.34 (6H, s), 1.65-1.72 (2H, m), 1.98 (3H, s), 2.05 (3H, s), 2.13 (3H, s), 2.34 (3H, s), 2.88 (2H, s), 3.27 (3H, s), 3.48 (2H, t, J = 6.0 H), 3.95-3.99 (2H, m), 5.87 (2H, brs), 7.11 (1H, d, J = 7.6 Hz), 7.28 (1H, t, J = 7.6 Hz), 7.35 (1H, d, J = 7.6 Hz), 7.74 (1H, s), 8.69 (1H, s); MS m/z 523 (M+H)+

Example 37: 6-amino-1-(2,3-dimethylphenyl)-5-(6-hydroxy-2,2,5,7-tetramethyl-3-oxaindan-4-yl)aminocarbonyl-3-(2,2,2-trifluoroethyl)uracil

The title compound was obtained by the same process as in Example 1.
1H NMR (DMSO-d6)δ 1.34 (6H, s), 1.99 (3H, s), 2.06 (3H, s), 2.12 (3H, s), 2.35 (3H, s), 2.88 (2H, s), 4.57-4.69 (2H, m), 6.10 (2H, brs), 7.15 (1H, d, J = 7.6 Hz), 7.28-7.32 (1H, m), 7.38 (1H, d, J = 7.5 Hz), 7.76 (1H, s), 8.77 (1H, s)

Evaluation Example 1: Inhibitory effect on dermatitis induced by picryl chloride

In order to verify the inflammation-inhibiting effect of the uracil derivative of the present invention, its effect on dermatitis induced by picryl chloride, a model of typical type IV allergic inflammation was evaluated by adopting the method of Asherson et al. (Immunology, 15, 405 (1968)). On the abdominal skin of each of ICR strain male mice was applied 0.1 ml of a 7% (w/v) picryl chloride/acetone solution to induce sensitization. On the seventh days after the sensitization, 0.02 ml of a 1% (w/v) picryl chloride/acetone solution was applied on the ear of each mouse to cause an allergic reaction. Immediately after this causing, 0.04 ml of acetone (a control) or a 0.25% (w/v) test compound/acetone solution was applied on the ear. Twenty-four hours after the causing, the thickness of the ear was measured and the dermatitis-inhibiting effect of the test compound was evaluated by employing the difference between the thickness of the ear 24 hours after the causing and that before the causing as an indication. The thickness of the ears of the mice treated with the test compound was compared with the thickness of the ears of mice on which 0.04 ml of a 2.5% (w/v) solution of the comparative compound 1 (the following structure) disclosed in Japanese Patent No. 3093170, as a comparative example, in acetone had been applied:

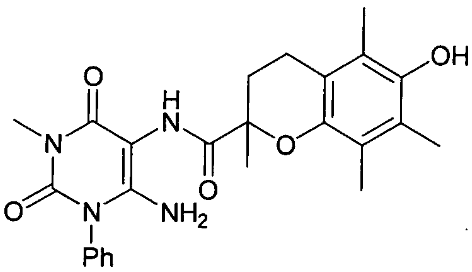

Comparative compound 1

From the difference of the ear thickness before and after the causing of the allergic reaction in the case of each group, the relative degree of efficacy of the test compound in the inhibition of ear enlargement was calculated according to the following equation by comparison with a group treated with the comparative compound in an amount of 10 times that of the test compound:

$$\text{Degree of efficacy} = \frac{\left(\text{Difference of ear thickness in the case of control group}\right) - \left(\text{Difference of ear thickness in the case of group treated with test compound}\right)}{\left(\text{Difference of ear thickness in the case of control group}\right) - \left(\text{Difference of ear thickness in the case of comparative group}\right)}$$

As a result, it was found that as shown in Table 19, the degree of efficacy of the uracil derivatives of the present invention was about 1. This fact indicates that the uracil derivatives of the present invention exhibit enlargement-inhibiting effect substantially equal to that obtained by the application of the compound as comparative example in an amount of 10 times that of each uracil derivative of the present invention. Therefore, it was revealed that the uracil derivatives of the present invention are superior to the compound as comparative example in anti-inflammatory effect on type IV allergic inflammation.

Table 19

| Test compound | Degree of efficacy |
|---|---|
| Example 4 | 0.9 |
| Example 5 | 1.1 |

Evaluation Example 2: Inhibitory effect on dermatitis induced by picryl chloride

The effect on the animal model in Evaluation Example 1 was verified by oral administration. ICR strain male mice were sensitized in the same manner as in Evaluation Example 1, and on the seventh days after the sensitization, 0.02 ml of a 1% (w/v) picryl chloride/acetone solution was applied on the ear of each mouse to cause an allergic reaction. Each test compound was suspended in a 0.5% CMC-Na solution and orally administered in a dose of 10 mg/kg one hour before the causing of the allergic reaction. As a comparative example, the above-mentioned comparative compound 1 disclosed in Japanese Patent No. 3093170 was orally administered in a dose of 100 mg/kg to make a comparison.

As a result, it was found that as shown in Table 20, the degree of efficacy of the uracil derivatives of the

present invention was 1.0 to 1.6. This fact indicates that the uracil derivatives of the present invention exhibit enlargement-inhibiting effect substantially equal to that obtained by the administration of the compound as comparative example in an amount of 10 times that of each uracil derivative of the present invention. Therefore, it was revealed that the uracil derivatives of the present invention are superior to the compound as comparative example in anti-inflammatory effect on type IV allergic inflammation.

Table 20

| Test compound | Degree of efficacy |
|---|---|
| Example 10 | 1.6 |
| Example 14 | 1.0 |
| Example 20 | 1.2 |
| Example 22 | 1.3 |
| Example 29 | 1.4 |
| Example 30 | 1.6 |

Evaluation Example 3: Inhibitory effect on the itch-related behavior of mice induced by substance P

It has been reported that in the lesion part of a patient with atopic dermatitis, there are observed an increase in nerve fiber containing substance P (SP), a nerve peptide (Tobin D et al., J Allergy Clin immunol, 90, 613-22(1992)) and an increase in reactivity with SP (Gianetti A et al. Br J Dermatol, 121, 681-8(1989)). On the other hand, it has been reported that when SP is administered to the cervicodorsal part of a mouse, itch-related behavior is induced (Kuraishi Y et al. Eur J Pharmacol, 275, 229-33(1995)) and is suppressed by a certain antiallergic agent (Inagaki N et al. Eur J Pharmacol, 400, 73-9(2000)). Therefore, in order to verify the itch-inhibiting effect of the uracil derivatives of the present invention, the itch-inhibiting effect was evaluated by employing the effect of the uracil derivatives on itch-related behavior induced by SP, as an indication. Each of BALB/c strain male mice was previously transferred to a cage for observation and allowed to acclimate to an observation environment, and then SP was subcutaneously administered (200 µg/mouse) to the cervicodorsal part of the mouse. After the administration, the mouse was returned to the cage for observation and the frequency of its itch-related behavior was measured for 60 minutes after the return. Each test drug was suspended in a 0.5% CMC-Na solution and orally administered in a dose of 10 mg/kg 30 minutes before the administration of SP. For evaluating the itch-inhibiting effect, the inhibition rate of the test drug was calculated as an indication as follows by comparison with a group treated with a 0.5% CMC-Na solution. As a comparative example, an antiallergic agent Oxatomide, which is considered as an agent having itch-inhibiting effect, was administered in a dose of 3 times that of the test drug (30 mg/kg).

$$\text{Inhibition rate} = \frac{\left(\begin{array}{l}\text{Frequency in the}\\ \text{case of group}\\ \text{treated with 0.5\%}\\ \text{CMC-Na solution}\end{array}\right) - \left(\begin{array}{l}\text{Frequency in the}\\ \text{case of group}\\ \text{treated with test}\\ \text{drug}\end{array}\right)}{\left(\begin{array}{l}\text{Frequency in the}\\ \text{case of group}\\ \text{treated with 0.5\%}\\ \text{CMC-Na solution}\end{array}\right)} \times 100$$

As a result, it was found that as shown in Table 21, the degree of the itch-inhibiting effect of the uracil derivatives of the present invention is equal to or larger than that of the antiallergic agent Oxatomide.

Table 21

| Test compound | Inhibition rate (%) |
|---|---|
| Oxatomide 30 mg/kg | 39 |
| Example 2 10 mg/kg | 71 |
| Example 5 10 mg/kg | 53 |

Formulation Example 1: Water-soluble ointment

A water-soluble ointment having the following composition was prepared by a conventional process:

| Ingredients | Content per 2 g of ointment |
|---|---|
| Compound of Example 4 | 40 mg |
| Poly(ethylene glycol) 400 | 1372 mg |
| Poly(ethylene glycol) 4000 | 588 mg |

Formulation Example 2: Tablets for internal use

Tablets for internal use having the following composition was prepared by a conventional process:

| Ingredients | Amount (mg/tablet) |
|---|---|
| Compound of Example 5 | 100 |
| Lactose | 353 |
| Carboxymethyl cellulose calcium | 30 |
| Hydroxypropyl cellulose | 7 |
| Magnesium stearate | 5 |
| Crystalline cellulose | 5 |
| Total | 500 mg |

INDUSTRIAL APPLICABILITY

The uracil derivatives of the present invention have not_only a marked inflammation-inhibiting effect on allergic inflammation, in particular, type IV allergic inflammation, but also itch-inhibiting effect. The uracil derivatives of the present invention suppress type IV allergic inflammation effectively and hence are useful as a therapeutic agent for allergic diseases, in particular, type IV allergic diseases. They can also suppress itch and hence are useful also as anti-itch agent. They are very useful particularly as a therapeutic agent for atopic dermatitis from the viewpoint of both anti-inflammation effect and anti-itch effect. In addition, since they are absorbed through skin when administered as an external preparation, they are useful for treating skin diseases such as atopic dermatitis, contact dermatitis, psoriasis and the like. Furthermore, said uracil derivatives are advantageous in that they are nonsteroidal substances and hence do not exhibit any adverse effect similar to that of steroids.

**Claims**

**1.** A uracil derivative represented by the general formula (I):

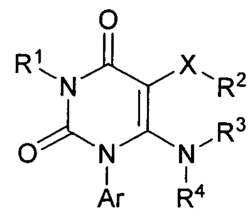

(I)

wherein X represents a group selected from NHCO, $NHCH_2$, CO, CONH and $CH_2NH$; $R^1$ represents a hydrogen atom or a substituted or unsubstituted alkyl group of 1 to 6 carbon atoms; $R^2$ represents the general formula (II) or (III):

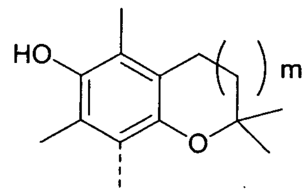

(II)

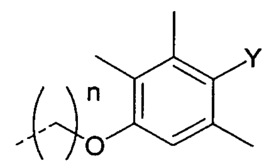

(III)

wherein m is 0 or 1, n is an integer of 1 to 3, Y is OH or $NH_2$, and each dotted line indicates a bonding position, provided that when $R^2$ represents the general formula (III), X represents NHCO or $NHCH_2$; $R^3$ and $R^4$ independently represent a hydrogen atom or an alkyl group of 1 to 6 carbon atoms; and Ar represents a phenyl group substituted by alkyl groups of 1 to 6 carbon atoms at the o- and m-positions, a substituted or substituted heteroaryl group or a bicyclic aromatic group, or a pharmaceutically acceptable salt thereof.

**2.** A uracil derivative or a pharmaceutically acceptable salt thereof according to claim 1, wherein $R^2$ represents the general formula (II) in the general formula (I).

**3.** A uracil derivative or a pharmaceutically acceptable salt thereof according to claim 1, wherein $R^2$ represents the general formula (III) in the general formula (I).

**4.** A uracil derivative or a pharmaceutically acceptable salt thereof according to claim 1 or 2, wherein Ar represents a phenyl group substituted by alkyl groups of 1 to 6 carbon atoms at the o- and m-positions in the general formula (I).

**5.** A uracil derivative or a pharmaceutically acceptable salt thereof according to claim 1 or 2, wherein Ar represents a substituted or unsubstituted heteroaryl group in the general formula (I).

**6.** A uracil derivative or a pharmaceutically acceptable salt thereof according to claim 1 or 2, wherein Ar represents a bicyclic aromatic group in the general formula (I).

**7.** A uracil derivative or a pharmaceutically acceptable salt thereof according to any one of claims 1 to 6, wherein X represents NHCO in the general formula (I).

**8.** A pharmaceutical composition for the treatment of allergic diseases comprising a uracil derivative or a pharmaceutically acceptable salt thereof according to any one of claims 1 to 7 as an active ingredient.

**9.** A pharmaceutical composition for the treatment of pruritus comprising a uracil derivative or a pharmaceutically acceptable salt thereof according to any one of claim 1 to 7 as an active ingredient.

## INTERNATIONAL SEARCH REPORT

International application No.
PCT/JP03/11859

A. CLASSIFICATION OF SUBJECT MATTER
Int.Cl$^7$ C07D239/54, 405/06, 405/12, 405/14, A61K31/513, A61P17/04, 37/08

According to International Patent Classification (IPC) or to both national classification and IPC

B. FIELDS SEARCHED

Minimum documentation searched (classification system followed by classification symbols)
Int.Cl$^7$ C07D239/54, 405/06, 405/12, 405/14, A61K31/513

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| A | JP 8-27121 A (Japan Energy Corp.), 30 January, 1996 (30.01.96), Full text (Family: none) | 1-9 |
| A | TOBE, M.; ISOBE, Y.; GOTO, Y.; OBARA, F.; TSUCHIYA, M.; MATSUI, J.; HIROTA, K.; HAYASHI, H., Synthesis and biological evaluation of CX-659S and its related compounds for their inhibitory effects on the delayed-type hypersensitivity reaction, Bioorganic & Medicinal Chemistry (2000), 8(8), 2037-2047 | 1-9 |

☐ Further documents are listed in the continuation of Box C.  ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 November, 2003 (04.11.03) | 18 November, 2003 (18.11.03) |
| Name and mailing address of the ISA/ Japanese Patent Office | Authorized officer |
| Facsimile No. | Telephone No. |

Form PCT/ISA/210 (second sheet) (July 1998)